# EUROPEAN PATENT APPLICATION

(11) **EP 4 555 985 A1**
(43) Date of publication of application: **21.05.2025**
(21) Application number: 23926428.6
(22) Date of filing: 16.11.2023
(51) Int. Cl.: A61F 13/49, A61F 13/53, A61F 13/532, A61F 13/533, A61F 13/535, A61F 13/536

(54) **ABSORBENT ARTICLE**

(30) Priority: 03.03.2023 JP 2023032724
(71) Applicant: Daio Paper Corporation, Ehime 799-0492 (JP)
(72) Inventor: YAMASHITA, Yuichi, Shikokuchuo-shi, Ehime 799-0113 (JP); TAKAGI, Yurika, Shikokuchuo-shi, Ehime 799-0113 (JP)
(74) Representative: Williams Powell
(86) International application number: PCT/JP2023/041206
(87) International publication number: WO 2024/185222

(57) **Abstract**

An absorbent article of which grainy texture of high-absorbent polymer particles therein is hard to be felt, is provided. This is achieved by an absorbent article having an absorbent element containing an absorber body and a wrapping nonwoven sheet wrapping the absorber body. The absorber body has a layer formed by mixing and accumulating pulp fibers and high-absorbent polymer particles, the absorbent element has grooves extending continuously in a lattice pattern, each forming a dent from the top face of the absorbent element into the absorber body and compressed in the thickness direction to form a bottom part. The grooves have crossing zones, and non-crossing zones each located between two adjacent crossing zones. At least partly over the top face of the absorbent element, the thickness of the bottom part is larger stepwise or continuously from the middle in the longitudinal direction of each non-crossing zone toward the adjacent crossing zones.

## Description

### FIELD OF ART

The present invention relates to an absorbent article, such as a disposable diaper.

### BACKGROUND ART

Absorbent articles in general have an absorber body therein, which has been formed by mixing and accumulating pulp fibers and high-absorbent polymer particles. Such an absorber body, for improved shape-retainability during and after its manufacture, is usually wrapped in a wrapping sheet of crepe paper or the like, to form an absorbent element, which is contained in an absorbent article (see, e.g., Patent Publications 1 and 2).

The absorbent element of an absorbent article, which is held between the legs of a wearer and subjected to forces in various directions from its sides opposed in the width direction with the motion of the legs in walking or the like, is required to fit well in the crotch section, while it is also required to have shape-retainability for keeping the absorber body from being deformed by distortion, cleavage, or the like. The absorbent element of a diaper is, naturally, required to have ensured absorption performance in the crotch section.

A technique known to improve shape-retainability of an absorber body is to provide grooves in a lattice pattern or the like, each having a bottom part formed by compressing an absorbent element in the thickness direction through embossing or the like (see, e.g., Patent Publications 1 and 2). However, in forming the compressed areas, with the wrapping sheet for wrapping the absorber body formed of crepe paper, the crepe paper may break at the edges of the compressed areas or between adjacent compressed areas. The crepe paper in a wet state, in which excrete liquid is absorbed, has a lowered strength and tends to break. Such breakage of the wrapping sheet is not preferable as the high-absorbent polymer particles may escape through the breakage out to the surface of the absorbent article.

It is also known to use fine-textured nonwoven sheet (referred to as wrapping nonwoven fabric hereinbelow) as the wrapping sheet. The wrapping nonwoven sheet, however, causes a problem of grainy texture derived from the high-absorbent polymer particles, felt when the bottom part of a groove formed in the absorbent element surface is traced with a finger. This grainy texture, when felt by the user, may deteriorate wearing comfort or damage product image, which is not preferred.

### PRIOR ART PUBLICATION

### PATENT PUBLICATION

Patent Publication 1: JP 2021-000236 A
Patent Publication 2: JP 6380454 B

### SUMMARY OF THE INVENTION

### PROBLEM TO BE SOVED BY THE INVENTION

It is therefore an object of the present invention to provide an absorbent article with wrapping nonwoven sheet, of which grainy texture derived from the high-absorbent polymer particles is hard to be felt by the user.

### MEANS FOR SOLVING THE PROBLEM

The absorbent article which solves the problem discussed above is as follows.

### <First Aspect>

An absorbent article having a crotch section, and front and back sections extending forward of and backward of the crotch section, respectively, the absorbent article including:
an absorbent element including an absorber body arranged in a position including the crotch section, and a wrapping nonwoven sheet wrapping the absorber body,
wherein the absorber body has a layer formed by mixing and accumulating pulp fibers and high-absorbent polymer particles,
wherein the absorbent element has grooves extending continuously in a lattice pattern, each forming a dent from a top face of the absorbent element down into the absorber body and compressed in a thickness direction to form a bottom part, and
wherein the grooves have crossing zones, and non-crossing zones each located between two adjacent crossing zones, and at least partly over the top face of the absorbent element, a thickness of the bottom part is larger stepwise or continuously from a middle in a longitudinal direction of each non-crossing zone toward the adjacent crossing zones.

### <Function and Effect>

Where the wrapping nonwoven sheet is used, formation, in the top face of the absorbent element, of the grooves compressed in the thickness direction to form a bottom part, causes compression of the wrapping nonwoven sheet and the absorber body in the thickness direction at the bottom part of the grooves, resulting in relative increase in the amount of the high-absorbent polymer particles at or near the top face of the absorber body, and hardly movable retention of the high-absorbent polymer particles at or near the top face the absorber body, which is rigid due to densification and decrease in cushioning property in the thickness direction of the wrapping nonwoven sheet and the absorber body. Such a rigid absorber body is covered with the thinned wrapping nonwoven sheet. Thus, when the bottom part of a groove is traced with a finger, grainy texture derived from the high-absorbent polymer particles is felt.

Here, the high-absorbent polymer particles posing troubles are located at the bottom part of the grooves, which is far from the skin of the wearer. That is, the high-absorbent polymer particles are located where they are hard to be touched by the user directly or indirectly via other sheets, so that, basically, the texture of the high-absorbent polymer particles is hard to be felt by the user. Irrespective of this, when the grooves are formed in a lattice pattern, the wrapping nonwoven sheet, at a corner of each crossing zone at the bottom parts of the grooves, is pulled in two directions, which makes the thickness of the absorbent element at that corner particularly thin, resulting in the crossing zone being easier to be brought into direct or indirect contact with the skin, compared to the non-crossing zones.

In contrast, according to the absorbent article of the present invention, (a) with the thickness of the bottom part being larger stepwise or continuously from the middle in the longitudinal direction of each non-crossing zone toward the adjacent crossing zones (that is, the degree of compression and the density of the bottom part are lower from the middle in the longitudinal direction of each non-crossing zone toward the adjacent crossing zones), decrease in thickness of the absorbent element at a corner of each crossing zone of the bottom parts of the grooves is reduced, so that the crossing zones of the bottom parts of the grooves are hardly touched by the skin of the user. In this way, the grainy texture of the high-absorbent polymer particles is harder to be felt by the user, compared to the case with the bottom part of each groove having generally a uniform thickness. Further, (b) at each crossing zone of the bottom parts of the grooves, which is more likely to be touched by the skin of the user, increase in the amount of the high-absorbent polymer particles at or near the top face of the absorber body is reduced, while densification and decrease in cushioning property in the thickness direction are also reduced, so that even when the crossing zones of the bottom parts of the grooves are brought into contact with the skin of the user, the grainy texture of the high-absorbent polymer particles is slight and thus still harder to be felt by the user.

The grooves extend continuously in a lattice pattern, each forming a dent from the top face of the absorbent element down into the absorber body and compressed in the thickness direction to form the bottom part, so that liquid-diffusibility and shape-retainability of the absorber body may be ensured.

### <Second Aspect>

The absorbent article according to the first aspect,
wherein a basis weight of the pulp fibers in the absorber body is 100 to 450 g/m²,
wherein a ratio by weight of the pulp fibers to the high-absorbent polymer particles in the absorber body is 30:70 to 70:30,
wherein a maximum thickness of the absorbent element is 4 to 35 mm,
wherein a thickness of the bottom part in the middle in the longitudinal direction of each non-crossing zone is 15 to 35% the maximum thickness of the absorbent element, and
wherein a thickness of the bottom part in each crossing zone is 1.4 to 6 times the thickness of the bottom part in the middle in the longitudinal direction of the non-crossing zone.

### <Function and Effect>

The material composition of the absorber body and the degree of compression of the bottom part of each groove may suitably be decided, and preferably within the ranges of this aspect.

### <Third Aspect>

The absorbent article according to the second aspect,
wherein a width of the bottom part is 1 to 3 mm, and
wherein a pattern of the grooves is a rhombic lattice pattern composed of first lines, each extending obliquely at 40 to 50° clockwise with respect to a front-back direction in plan view, and second lines, each extending obliquely at 40 to 50° counterclockwise with respect to the front-back direction in plan view.

### <Function and Effect>

The dimensions and shape of the grooves or the like may suitably be decided, and the grooves formed in a rhombic lattice pattern as in the present aspect produce not only excellent shape-retainability and flexibility, but also excellent liquid-diffusibility, of the absorber body, which is preferable. The grooves formed in the pattern according to this aspect particularly contribute to reduction of decrease in thickness of the absorbent element at a corner of each crossing zone of the bottom parts of the grooves, which is preferable.

### <Fourth Aspect>

The absorbent article according to any one of the first to third aspects,
wherein the wrapping nonwoven sheet has one or a plurality of meltblown layers, and one or a plurality of protective layers,
wherein a basis weight of the wrapping nonwoven sheet is 10 to 17 g/m²,
wherein constituent fibers of each protective layer have an average fiber diameter of 10 to 25 µm,
wherein constituent fibers of each meltblown layer have an average fiber diameter of 5 µm or smaller,
wherein the wrapping nonwoven sheet has an air permeability of 25.5 to 70.0 cm³/cm²·s, as determined of a stack of five sheets thereof by Method A (Frazier Type Method) in compliance with JIS L 1096: 2010,
wherein the wrapping nonwoven sheet has an elongation of 20 to 100% in the front-back direction under normal conditions as determined in compliance with JIS L 1913: 2010, and
wherein the wrapping nonwoven sheet has an elongation of 20 to 110% in a width direction under normal conditions as determined in compliance with JIS L 1913: 2010.

### <Function and Effect>

In the absence of stretchability of the wrapping nonwoven sheet, grooves of a sound structure are hard to be formed, and flexibility of the absorbent element is low. Further, sparse fabric construction of the wrapping nonwoven sheet may lead to easy escape of the high-absorbent polymer particles. Thus, it is preferred that the wrapping nonwoven sheet is sufficiently thin and stretchable, and has a dense meltblown layer, as in the present aspect. With uniform grooves and such wrapping nonwoven sheet, the grainy texture of the high-absorbent polymer particles is readily felt by the user. With the features (a) and (b) discussed above, the absorbent element has improved shape-retainability and flexibility of the absorber body, while the grainy texture of the high-absorbent polymer particles may be kept from being felt by the user.

Note that the air permeability may be an index of ease of escape of the high-absorbent polymer particles.

### <Fifth Aspect>

The absorbent article according to the fourth aspect, wherein the basis weight of the wrapping nonwoven sheet is 15 to 17 g/m².

### <Function and Effect>

In the absence of stretchability of the wrapping nonwoven sheet, grooves of a sound structure are hard to be formed, and flexibility of the absorbent element is low. Further, sparse fabric construction of the wrapping nonwoven sheet may lead to easy escape of the high-absorbent polymer particles. Thus, it is preferred that the wrapping nonwoven sheet is sufficiently thin and stretchable, and has a dense meltblown layer, as in the present aspect. These are as discussed above.

However, forming, in the top face of the absorbent element, grooves in a lattice pattern, each having a compressed area as its bottom part compressed in the thickness direction, for the purpose of improving shape-retainability of the absorber body and liquid diffusibility in the front-back and the width directions of the absorbent element, may result in escape of the high-absorbent polymer particles, when the top face of the wrapping nonwoven sheet at or near a crossing zone of the grooves is subjected to frictional force. One of the conceivable major factors for this phenomenon may be that the wrapping nonwoven sheet, in or near each crossing zone, is pulled in two directions (the direction crossing one of the two adjacent grooves and the direction crossing the other of the grooves), which results in reduced thickness and enlarged interfiber gaps of the wrapping nonwoven sheet, compared to the remaining zones. Another of the conceivable major factors may be that, as a result of the absorber body being compressed in the thickness direction at or near the bottom part of each groove, the amount of the high-absorbent polymer particles at or near the top face of the absorber body (i.e., capable of escaping from the absorber body) is relatively increased.

In contrast, where the thickness of the bottom part is larger stepwise or continuously from the middle in the longitudinal direction of each non-crossing zone toward the adjacent crossing zones (that is, the degree of compression and the density of the bottom part are lower from the middle in the longitudinal direction of each non-crossing zone toward the adjacent crossing zones), compared to otherwise, elongation of the wrapping nonwoven sheet along both sides of each groove, as well as the resulting enlargement of the interfiber gaps and decrease in thickness, may be reduced from the middle in the longitudinal direction of each non-crossing zone toward the adjacent crossing zones. In this way, by combining a dense wrapping nonwoven sheet having a basis weight higher than a certain level as in the present aspect, and the varying thickness of the bottom part as discussed above, the high-absorbent polymer particles contained in the absorber body hardly escape through the wrapping nonwoven sheet even in or near the crossing zones.

### <Sixth Aspect>

The absorbent article according to any one of the first to fifth aspects, wherein the absorber body has a covering layer in which only the pulp fibers are accumulated, to cover a top face of the absorber body.

### <Function and Effect>

With the covering layer as in the present aspect, the high-absorbent polymer particles are hard to escape through the top face of the absorber body, which is preferable.

### <Seventh Aspect>

The absorbent article according to any one of the first to sixth aspects,
wherein the wrapping nonwoven sheet has an under section located on an underside of the absorber body, a first top section extending continuous from the under section and around a side edge to a top side of the absorber body, and a second top section extending continuous from the under section and around another side edge to the top side of the absorber body,
wherein the first top section and the second top section overlap with each other in a layered section, and
wherein all of the grooves are formed in the layered section.

### <Function and Effect>

With the structure as in the present aspect, the high-absorbent polymer particles may be kept from escaping through the top face of the absorbent element.

### <Eighth Aspect>

The absorbent article according to any one of the fifth to seventh aspects, wherein a middle region other than opposed lateral sides in the width direction of the absorbent element is devoid of any covering with another member all along the front-back direction to expose the wrapping nonwoven sheet on a top face of the absorbent article.

### <Function and Effect>

As discussed above, since the high-absorbent polymer particles may be kept from escaping through the wrapping nonwoven sheet, members provided in conventional absorbent articles, such as a top sheet for covering the top face of the absorbent element, may be omitted, which allows significant cost reduction.

### EFFECT OF THE INVENTION

According to the present invention, advantages are provided, such as provision of an absorbent article with wrapping nonwoven sheet, of which grainy texture derived from the high-absorbent polymer particles is hard to be felt by the user.

### BRIEF DESCRIPTION OF THE DRAWINGS

Fig. 1 is a plan view of an underpants-type disposable diaper in its spread state, showing its internal face.
Fig. 2 is a plan view of the underpants-type disposable diaper in its spread state, showing its external face.
Fig. 3 is a cross-sectional view taken along lines 2-2 in Fig. 1.
Fig. 4 is a cross-sectional view taken along lines 3-3 in Fig. 1.
Fig. 5(a) is a sectional view taken along lines 4-4 in Fig. 1, and Fig. 5(b) is a sectional view taken along lines 5-5 in Fig. 1.
Fig. 6 is a perspective view of the underpants-type disposable diaper.
Fig. 7 is a plan view of the inner member in its spread state, showing its external face together with the outline of the outer members.
Fig. 8 is a plan view of the absorber body, showing its top face together with the outline of the wrapping sheet.
Fig. 9 is a sectional view of the absorbent element.
Fig. 10 is a plan view of the absorbent element.
Fig. 11 is an enlarged plan view of a relevant part of the top face of the absorbent element.
Fig. 12(a) is a sectional view taken along lines 7-7 in Fig. 11, and Figs. 12(b) to 12(d) are sectional views corresponding to the sectional view taken along lines 7-7 in Fig. 11.

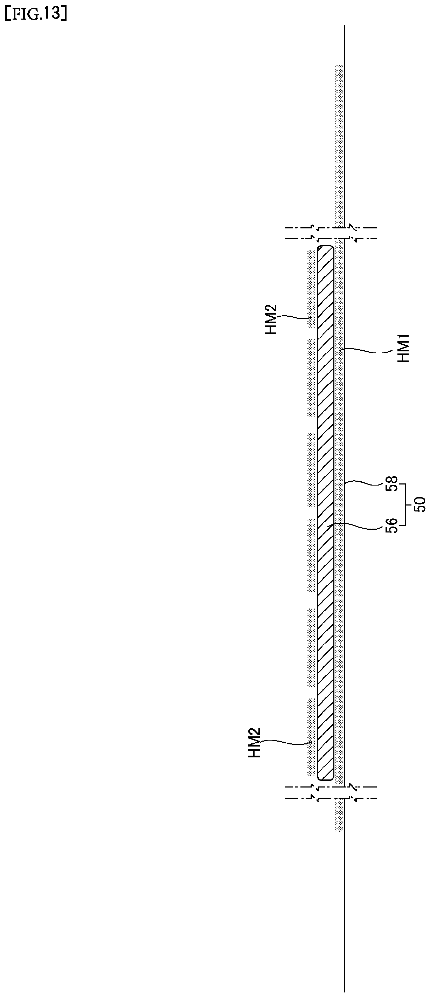

Fig. 13 is a sectional view of the absorbent element before the absorber body is wrapped in the wrapping sheet.

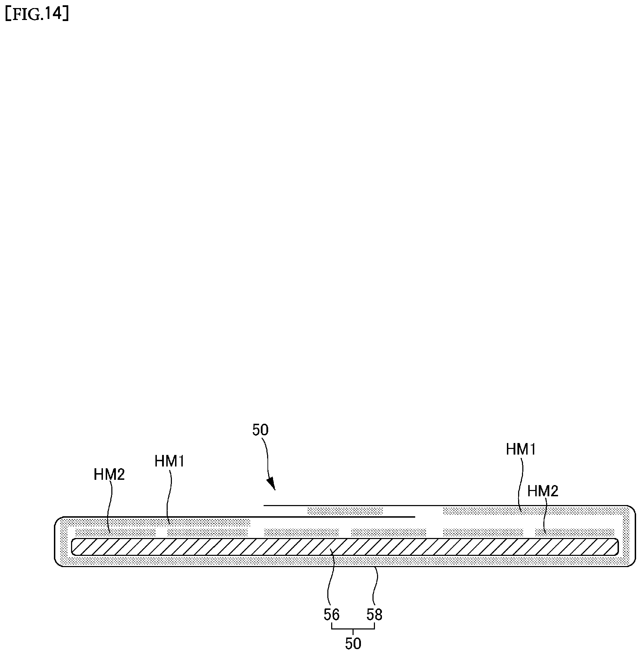

Fig. 14 is a sectional view of the absorbent element formed by wrapping the absorber body with the wrapping sheet, before the grooves are formed therein.
Fig. 15 is a sectional view of another absorbent element.
Fig. 16 illustrates other absorbent elements in plan view.
Fig. 17 is a cross-sectional view of another underpants-type disposable diaper corresponding to the cross-sectional view taken along lines 2-2 in Fig. 1.
Fig. 18 is a cross-sectional view of another underpants-type disposable diaper corresponding to the cross-sectional view taken along lines 3-3 in Fig. 1.
Fig. 19 is a cross-sectional view of another absorbent element.
Fig. 20 illustrates other absorber bodies in plan view.
Fig. 21 is a schematic view illustrating a method for producing an absorbent element.
Fig. 22 illustrates protrusions of anvil rolls in perspective.
Fig. 23 is a schematic view of testing apparatus.
Fig. 24 is an explanatory view of only a relevant part of a rubbing pattern.

### MODES FOR CARRYING OUT THE INVENTION

An underpants-type disposable diaper, as an examples of a disposable diaper, will now be discussed in detail with reference to the attached drawings. Note that components adjacent to each other in the thickness direction may be fixed or joined as necessary in the same way as in known diapers, at locations other than the fixed or joined areas to be discussed below. Dotted pattern regions in the sectional views represent an adhesive, such as a hot melt adhesive, as means of the fixing or joining. The hot melt adhesive may be applied by any known manner, such as slot coating, continuous or intermittent linear bead coating, spray coating in spiral, Z-shaped, wave, or the like patterns, or pattern coating (transfer of a hot melt adhesive by relief printing). In place of or in addition to this, on fixing portions of elastic members, a hot melt adhesive may be applied to the external surface of the elastic members for fixing to adjacent members. Examples of the hot melt adhesive include, but not limited to, EVA-based, adherent rubber-based (elastomer-based), polyolefin-based, and polyester/polyamide-based adhesives. The fixing or joining means for joining components may alternatively be material melt-bonding, such as heat sealing or ultrasonic sealing. Components adjacent to each other in the thickness direction are fixed or joined in an intermittent pattern, where liquid permeability in the thickness direction is required. For example, for such intermittent fixing or joining with a hot melt adhesive, intermittent pattern coating in a spiral, Z, wave, or the like shape may preferably be employed. For application in an area wider than the application width of one nozzle, intermittent pattern coating in a spiral, Z, wave, or the like shape may be employed with or without space in the width direction. The joining means for joining components may be material melt-bonding, such as heat sealing or ultrasonic sealing.

As the nonwoven fabric in the description hereinbelow, commonly known nonwoven fabric may suitably be used depending on the parts or purposes. Examples of the constituent fibers of the nonwoven fabric include, but not limited to, synthetic fibers, such as polyolefin-based, e.g., polyethylene or polypropylene, polyester-based, or polyamide-based fibers (including not only single component fibers, but also composite fibers, such as of core/sheath type), as well as regenerated fibers, such as rayon or cupra, or natural fibers, such as cotton, and also mixtures thereof. For improved flexibility of the nonwoven fabric, the constituent fibers may preferably be crimped fibers. The constituent fibers of the nonwoven fabric may also be hydrophilic fibers (including those rendered hydrophilic with hydrophilizers), hydrophobic fibers, or water-repelling fibers (including those rendered water-repelling with water repellents). Further, nonwoven fabric may generally be categorized into short fiber nonwoven, long fiber nonwoven, spunbonded nonwoven, melt blown nonwoven, spunlace nonwoven, thermal bonded (air through) nonwoven, needle-punched nonwoven, point-bonded nonwoven, composite nonwoven (including SMS or SMMS nonwoven fabric having one or more melt blown layers interposed between spunbonded layers), or the like nonwoven fabric, generally depending on the length of the fibers, method of forming the sheet, method of joining the fibers, or layered structure, and any of these nonwoven fabric may be used.

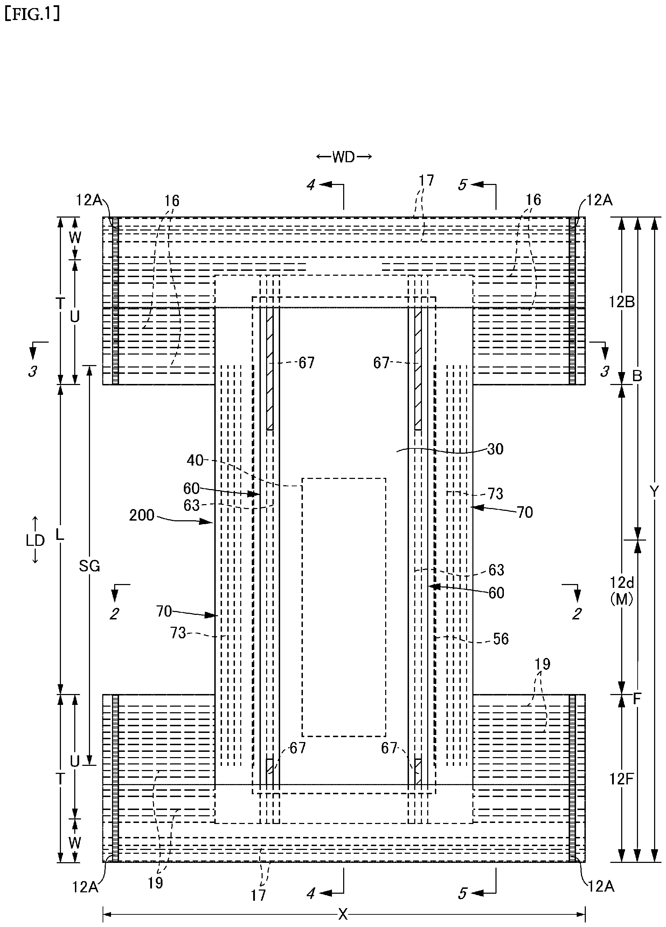

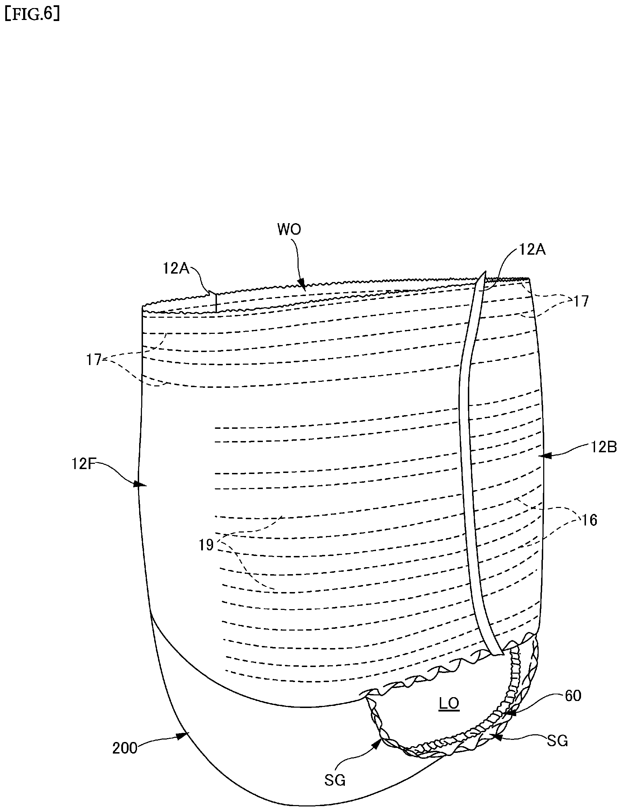

Figs. 1 to 6 show an embodiment of an underpants-type disposable diaper. The underpants-type disposable diaper according to the present embodiment has a rectangular front outer member 12F forming a front lower torso section, a rectangular back outer member 12B forming a back lower torso section, and an inner member 200 provided on the inner faces of the outer members 12F, 12B to extend from the front outer member 12F, via crotch section M, to the back outer member 12B. The front outer member 12F and the back outer member 12B are joined along their opposed lateral sides to form a pair of side seals 12A, to thereby define an opening with the front and back edges of the outer members 12F, 12B, which is a waist opening WO for passing the torso of the wearer therethrough, whereas a pair of leg openings LO is formed on either side in the width direction WD of the inner member 200, defined by the lower edges of the outer members 12F, 12B and the side edges of the inner member 200. The inner member 200 absorbs and holds bodily waste, such as urea, while the outer members 12F, 12B support the inner member 200 with respect to the body of a wearer. Reference sign Y refers to the entire length of the diaper in its spread state, with the front outer member 12F and the back outer member 12B not being joined with the pair of side seals 12A (the length in the front-back direction from the edge of the waist opening WO in the front body section F over to the edge of the waist opening WO in the back body section B), whereas reference sign X refers to the entire width of the diaper in its spread state.

The underpants-type disposable diaper according to the present embodiment has lower torso regions T defined as extents in the front-back direction over the side seals 12A (zones each extending in the front-back direction from the waist opening WO to the upper edge of a leg opening LO) and an intermediate section L defined as an extent in the front-back direction forming the leg openings LO (a region between the front-back region of the front body section F along the side seals 12A and the front-back region of the back body section B along the side seals 12A). The regions located in the lower torso regions T of the front outer member 12F and the back outer member 12B, i.e., the front lower torso section and the back lower torso section may each be divided conceptually into a waist zone W forming the edge zone of the waist opening and an under-waist zone U extending downward of the waist zone W. Usually, when the front lower torso section and the back lower torso section each has one or more boundaries between zones of different stretching stresses in the width direction WD (e.g., with elastic members of different finenesses or stretch rates), the zone on the waist opening WO side of the boundary closest to the waist opening WO is the waist zone W. In the absence of such boundaries, the zones of the front and back lower torso sections extending toward the waist opening WO beyond the absorber body 56 or the inner member 200 are the waist zones W. The length in the front-back direction of the waist zones may vary depending on the size of the product, and may suitably be decided. For example, each waist zone W may extend over 15 to 40 mm and each under-waist zone U may extend over 65 to 120 mm. On the other hand, the side edges of the intermediate section L are centrally narrowed in square U-shapes or curved shapes to fit the round-leg profile of the wearer, and the legs of the wearer are to be inserted here. Thus, underpants-type disposable diapers in the spread state have generally an hourglass shape as a whole.

### <Inner Member>

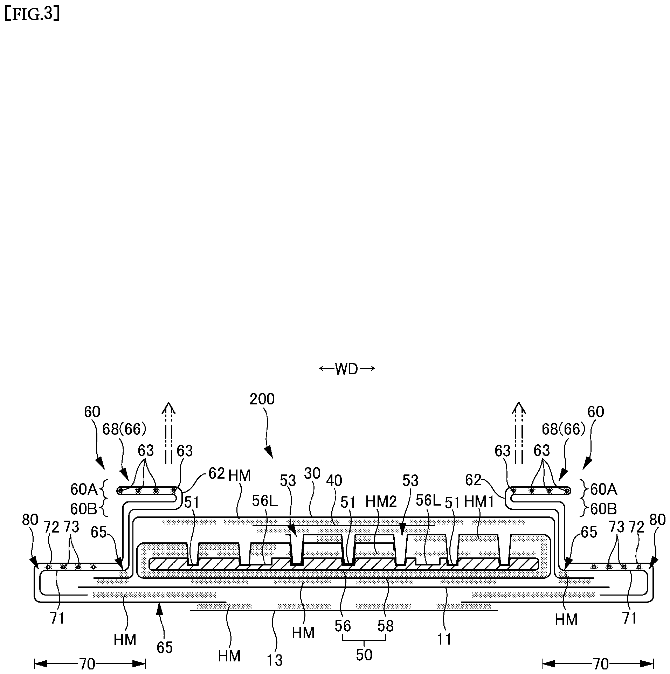

The inner member 200 may be in any shape and, in the illustrated embodiment, is in a rectangular shape having long sides forming the side edges. The inner member 200 has a top sheet 30 to be disposed on the side of the skin of the wearer, a liquid impervious sheet 11, and an absorbent element 50 interposed therebetween, as shown in Figs. 3 to 5, and assumes the absorbing and holding functions. Reference numeral 40 refers to an intermediate sheet (second sheet) interposed between the top sheet 30 and the absorbent element 50 so as to have the liquid permeated through the top sheet 30 promptly transferred to the absorbent element 50. Reference numeral 60 refers to a standup gather part extending from each lateral side of the inner member 200 so as to touch around a leg of the wearer in order to keep the bodily waste from leaking through the lateral sides of the inner member 200.

### <Top Sheet>

The top sheet 30 has a property to allow permeation of liquid therethrough, and may be, for example, perforated or non-perforated nonwoven fabric or perforated plastic sheet. Further, the top sheet 30 may be formed of a single sheet or a layered sheet prepared by bonding two or more sheets together. Similarly, the top sheet 30 may be formed, in the planar direction, of a single sheet or two or more sheets.

The top sheet 30 may be folded in each lateral side along the side edge of the absorbent element 50 to extend toward the underside thereof, or may not be folded to extend laterally beyond the side edge of the absorbent element 50. Alternatively, the overall width of the top sheet 30 may be shorter than the overall width of the absorbent element 50, so that the side edges of the top sheet 30 may be placed on the absorbent element 50 and the top face of the absorbent element 50 in its opposed lateral portions may be exposed on the top face of the diaper.

The top sheet 30 is preferably fixed to a member adjacent underside thereof by means of joining means through material melt-bonding, such as heat sealing or ultrasonic sealing, or a hot melt adhesive, for the purpose of keeping the top sheet 30 from displacing with respect to the member underside thereof. In the illustrated embodiment, the top sheet 30 is fixed to the top face of the intermediate sheet 40 and to the top face of part of the wrapping nonwoven sheet 58 that is located on the top side of the absorber body 56, by means of a hot melt adhesive applied to the underside of the top sheet 30.

### <Intermediate Sheet>

An intermediate sheet (also referred to as a second sheet) 40 may be provided, which has a higher liquid transmission rate compared to the top sheet 30, so as to have the liquid permeated through the top sheet 30 promptly transferred to the absorber body 56. This intermediate sheet 40 promptly transfers liquid to the absorber body 56 to improve the absorption performance of the absorber body 56, while it keeps the absorbed liquid from "back flowing" from the absorber body 56. The intermediate sheet 40 may be omitted. In this case, the top sheet 30 may also be omitted.

The intermediate sheet 40 may be made of, for example, a material similar to that of the top sheet 30, spunlace nonwoven fabric, spunbonded nonwoven fabric, SMS nonwoven fabric, pulp nonwoven fabric, mixed sheet of pulp and rayon, point-bonded nonwoven fabric, or crepe paper. Air through nonwoven fabric is particularly preferred for its bulkiness. For the air through nonwoven fabric, composite fibers having a core-in-sheath structure are preferably used, in which the resin used for the core may be polypropylene (PP), but may preferably be polyester (PET), which is highly rigid. The basis weight is preferably 17 to 80 g/m², more preferably 25 to 60 g/m². The fineness of the raw material fibers of the nonwoven fabric is preferably 2.0 to 10 dtex. For making the nonwoven fabric bulky, it is preferred to use eccentric fibers having the core off the center, hollow fibers, or eccentric and hollow fibers, as mixed fibers constituting all or part of the raw material fibers.

The intermediate sheet 40 in the illustrated embodiment is shorter than the absorber body 56 in width and positioned in the center, but may be provided all over the width of the absorber body 56. The dimension of the intermediate sheet 40 in the front-back direction may be the same as the overall length of the diaper, the same as the length of the absorbent element 50, or within a short length range around the area in which liquid is absorbed.

The intermediate sheet 40 is preferably fixed to a member adjacent underside thereof by means of joining means through material melt-bonding, such as heat sealing or ultrasonic sealing, or a hot melt adhesive, for the purpose of keeping the intermediate sheet 40 from displacing with respect to the member underside the intermediate sheet 40. In the illustrated embodiment, the intermediate sheet 40 is fixed to the top face of part of the wrapping nonwoven sheet 58 that is located on the top side of the absorber body 56, by means of a hot melt adhesive applied to the underside of the intermediate sheet 40.

### <Liquid-Impervious Sheet>

The liquid-impervious sheet 11 may be made of any material without particular limitation, for example, plastic film made of a polyolefin-based resin, such as polyethylene or polypropylene, laminated nonwoven fabric composed of nonwoven fabric and plastic film provided over the nonwoven fabric, a layered sheet composed of plastic film and nonwoven fabric or the like, layered and joined together. The liquid-impervious sheet 11 is preferably made of a material having both liquid-impermeability and moisture-permeability, which material is preferably used for preventing dampness. Such moisture-permeable plastic film may be microporous plastic film, which is widely used and obtained by adding an inorganic filler to a polyolefin-based resin, such as polyethylene or polypropylene, kneading the resulting mixture, forming the kneaded mixture into a sheet, and then uni- or biaxially drawing the sheet. Also, nonwoven fabric of microdenier fibers, nonwoven fabric that has been given an enhanced leak proof property by applying heat or pressure to minimize interfiber gaps, or sheets that have been rendered liquid-impervious without using plastic film through a process, such as coating with a highly water-absorbable resin or a hydrophobic resin or water repellent, may be used as the liquid-impervious sheet 11. For sufficient bonding strength in bonding to a cover nonwoven sheet 13 via a hot melt adhesive as will be discussed later, use of resin film is preferred.

The liquid-impervious sheet 11 may have a width that fits behind the absorbent element 50 as illustrated, or may wrap around the edges of the absorbent element 50 to extend to the opposed sides of the top sheet 30 of the absorbent element 50 for improved leak proof property. The width of such extensions may suitably be 5 to 20 mm on each of the right and left sides.

### <Absorbent Element>

The absorbent element 50 includes the absorber body 56 and a wrapping nonwoven sheet 58 that entirely wraps the absorber body 56.

### <Absorber Body>

The absorber body 56 is formed by mixing and accumulating pulp fibers and high-absorbent polymer particles. The pulp fibers may have a basis weight of, for example, about 100 to 450 g/m².

The high-absorbent polymer particles include not only "particles", but also "powders". The high-absorbent polymer particles may be those used in this type of disposable diapers as they are, and may preferably be particles 30 wt% or less of which, after sieving (five-minute shaking), remain on a 500 µm standard sieve (JIS Z8801-1: 2006) and particles 60 wt% or more of which, after sieving (five-minute shaking), remain on a 180 µm standard sieve (JIS Z8801-1: 2006).

Any material of the high-absorbent polymer particles may be used without particular limitation, and those having a water absorption of 40 g/g or more are preferred. The high-absorbent polymer particles may be starch-based, cellulose-based, or synthetic polymer-based, and starch-acrylic acid (salt) graft copolymers, saponified products of starch-acrylonitrile copolymers, cross-linked sodium carboxymethyl cellulose, acrylic acid (salt) polymers, or the like, may be used. The high-absorbent polymer particles may preferably be in ordinary powder or granular form, but particles in other forms may also be used.

The high-absorbent polymer particles having a water absorption speed of 70 seconds or less, particularly 40 seconds or less, may preferably be used. With too slow a water absorption speed, so-called back flow may likely to occur, in which liquid supplied into the absorber body 56 returns out of the absorber body 56.

The high-absorbent polymer particles may preferably have a gel strength of 1000 Pa or higher. With such property, when the high-absorbent polymer particles are formed into a bulky absorber body 56, stickiness after liquid absorption may effectively be limited.

The basis weight of the high-absorbent polymer particles may suitably be decided depending on the amount of absorption required in a use of the absorber body 56. Thus, it depends, but the basis weight may be 50 to 350 g/m². At a basis weight of the polymer less than 50 g/m², the amount of absorption may hardly be secured. At over 350 g/m², the effect may be saturated.

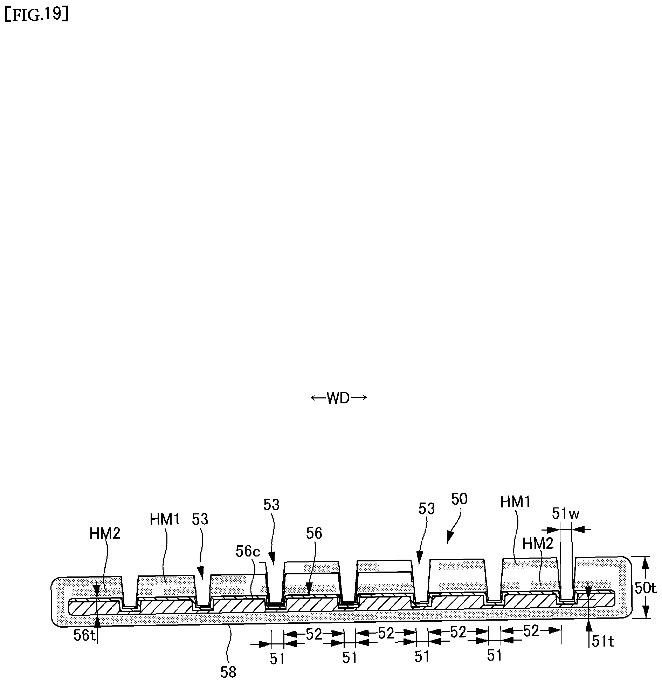

The ratio of the pulp fibers to the high-absorbent polymer particles in the absorber body 56 is not particularly limited, and may be 40:60 to 65:35 by weight. In particular, at a ratio of the pulp fibers to the high-absorbent polymer particles of 50:50 to 65:35 by weight, the absorber body 56 is excellent in shape retainability, absorption rate, ease of formation of grooves 53 to be discussed later, and the high-absorbent polymer particles hardly escape, which are preferred. Further, the high-absorbent polymer particles are preferably present generally uniformly throughout the absorber body 56. However, for withholding the high-absorbent polymer particles from escaping through the top face of the absorber body, the content of the high-absorbent polymer particles preferably decreases from the underside toward the top side of the absorber body stepwise or continuously. In particular, as shown in Fig. 19, a covering layer 56c in which only the pulp fibers are accumulated, is preferably provided to cover the top face of the absorber body 56. Even with this covering layer 56c formed only of the pulp fibers, the ratio of the fibers to the high-absorbent polymer particles is preferably within the above-discussed range.

The thickness 56t of the absorber body 56 is not particularly limited, and may be 3 to 15 mm.

It suffices that the absorber body 56 extends continuously from forward to backward of the crotch section M to include the crotch section M. In an underpants-type disposable diaper like this embodiment, the absorber body 56 extends in the front-back direction LD and in the width direction WD preferably up to or to the vicinity of the peripheral edges of the inner member 200. Reference sign 56X refers to the maximum width of the absorber body 56.

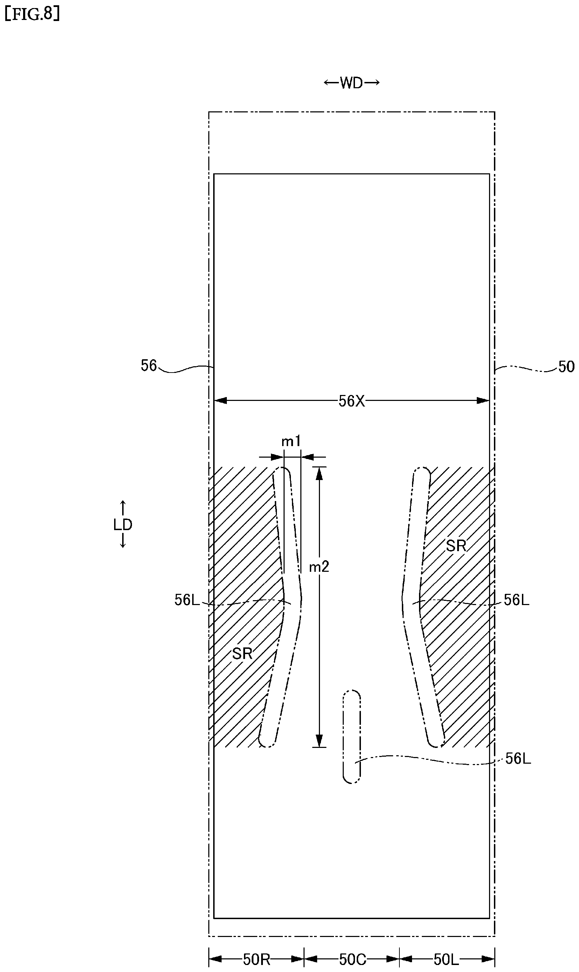

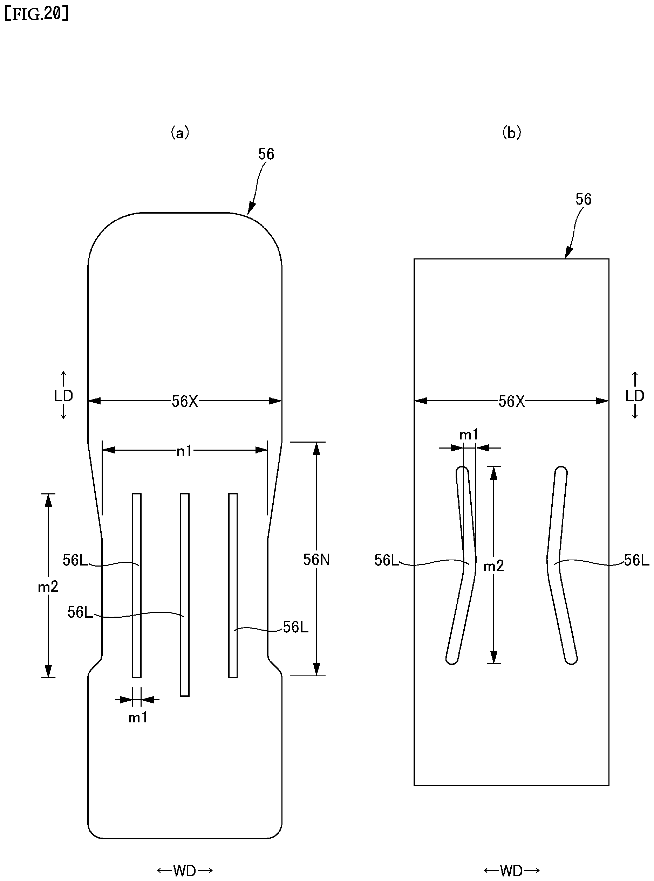

For assisting ensured amount of absorption in the crotch section M, the absorber body 56 may preferably be in a generally rectangular shape as in the embodiment illustrated in Fig. 8. Alternatively, as in the embodiment illustrated in Fig. 20(a), for improved fitting in the crotch section M, the width of the absorber body 56 may be narrowed in the crotch section M compared to the widths forward and backward of the crotch section M to impart a constricted shape to the absorber body 56. In this case, for assisting ensured amount of absorption in the crotch section M, it is preferred that the narrowest width n1 of the absorber body 56 in the crotch section M is 0.85 times or more of the maximum width 56X of the absorber body 56.

Note that, when the absorber body 56 has a constricted zone 56n as will be discussed later, the crotch section M refers to the extent in the front-back direction LD including this constricted zone 56n, whereas, when the absorber body 56 does not have the constricted zone 56n but the contour of the diaper in the spread state has a constricted zone as in the illustrated embodiment, the crotch section M refers to the extent in the front-back direction LD including this constricted zone of the contour (in the illustrated embodiment, between the front outer member 12F and the back outer member 12B), or where the absorber body 56 has neither of the constricted zones, the crotch section M refers to the extent in the middle in the front-back direction LD corresponding to 20 to 30% the entire length of the product. The extensions forward and backward of the crotch section M are forward and backward zones, respectively.

### <Lower-basis-weight area>

The absorber body 56 may have elongate lower-basis-weight areas 56L extending in the front-back direction LD on the lateral sides opposed in the width direction WD of the crotch section M or the like, as in the embodiment illustrated in Figs. 8 and 20(a), or may not have such lower-basis-weight areas 56L, as in the embodiment illustrated in Figs. 1 to 6. A lower-basis-weight area 56L denotes an area having a lower basis weight, and does not encompass an area which is only compressed in the thickness direction TD without any difference in basis weight, like the bottom part 51 of the groove 53 as will be discussed later. A lower-basis-weight area 56L may be in the form of a slit penetrating the absorber body 56 in the thickness direction, or may be in the form of a recess with smaller amounts of accumulated pulp fibers and high-absorbent polymer particles, as in the illustrated embodiment, which is preferred for ensuring amount of absorption. The recess may be formed in the top face or the under face of the absorber body 56. By providing the absorber body 56 with the lower-basis-weight areas 56L, flexure of the absorber body 56 along the lower-basis-weight areas 56L is promoted to improve fitting of the absorbent element 50 in the crotch section M. It suffices that the total basis weight of the pulp fibers and the high-absorbent polymer particles in the lower-basis-weight areas 56L is lower than the total basis weight of the pulp fibers and the high-absorbent polymer particles in the areas other than the lower-basis-weight areas 56L, and may be, for example, 0.1 to 0.5 times the total basis weight of the pulp fibers and the high-absorbent polymer particles in the areas other than the lower-basis-weight areas 56L.

A lower-basis-weight area 56L, as long as being elongate in the front-back direction LD, may extend linearly in the front-back direction LD, or may be curved laterally outwards with increasing proximity to its ends in the front-back direction LD, as in the illustrated embodiment. Further, each of the front and back ends of a lower-basis-weight area 56L may be in a suitable shape, for example, may be in the form of a straight line as in the embodiment shown in Fig. 20(a), may be protruded arcuately (semicircular or the like) as in the embodiment shown in Fig. 8, or may have rounded corners with a straight line therebetween, though not shown. The width m1 of a lower-basis-weight area 56L may suitably be decided, and may be, for example, 0.04 to 0.1 time the narrowest width n1 of the absorber body 56 in the crotch section M (entire width 56X where the absorber body 56 is rectangular). The width m1 of a lower-basis-weight area 56L may be constant or varied along its length direction. The dimension and arrangement in the front-back direction LD of a lower-basis-weight area 56L may suitably be decided. For example, the dimension m2 in the front-back direction LD of a lower-basis-weight area 56L may be 50 to 120%, more preferably 50 to 80% of the dimension in the front-back direction LD of the crotch section M. Further, a lower-basis-weight area 56L may be within the extent of the crotch section M, or may extend beyond the front and/or back ends of the crotch section M.

One lower-basis-weight area 56L may be provided on each side in the width direction WD of the crotch section M (both on the right and left sides) as well as in the center of the width direction WD as shown in Fig. 20(a), one lower-basis-weight area 56L may be provided only on each side in the width direction WD of the crotch section M as shown in Fig. 20(b), or only one lower-basis-weight area 56L may be provided along the center of the crotch section M, though not shown.

### <Groove>

The absorbent element 50 may preferably have grooves 53 extending continuously in a lattice pattern, each forming a dent from the top face of the absorbent element 50 down into the absorber body 56 and compressed in the thickness direction to form a bottom part 51, as shown in Figs. 3, 4, and 9. In this way, the grooves 53 in the top face of the absorbent element 50 extending continuously may improve liquid diffusibility and shape-retainability of the absorber body 56. The bottom parts 51 of the grooves 53 are high-density areas compressed by depressing (direct pressing) into a generally uniform thickness. The areas of the absorbent element 50 other than the bottom parts 51 are referred to as non-compressed areas 52 hereinbelow. The non-compressed areas 52 have a larger thickness and a lower density compared to those of the bottom parts 51, but even in the non-compressed areas, the absorber body 56 and the wrapping nonwoven sheet 58 near the bottom parts 51 are dragged by the deformation for the bottom parts 51 to have increased density with increasing proximity to the bottom parts 51. The non-compressed areas 52, as far as having a larger thickness and a lower density compared to those of the bottom parts 51, may be compressed overall in the thickness direction during, before, or after the compression for forming the bottom parts 51. The shape and arrangement of the non-compressed areas 52 are decided depending on the shape and arrangement of the bottom parts 51.

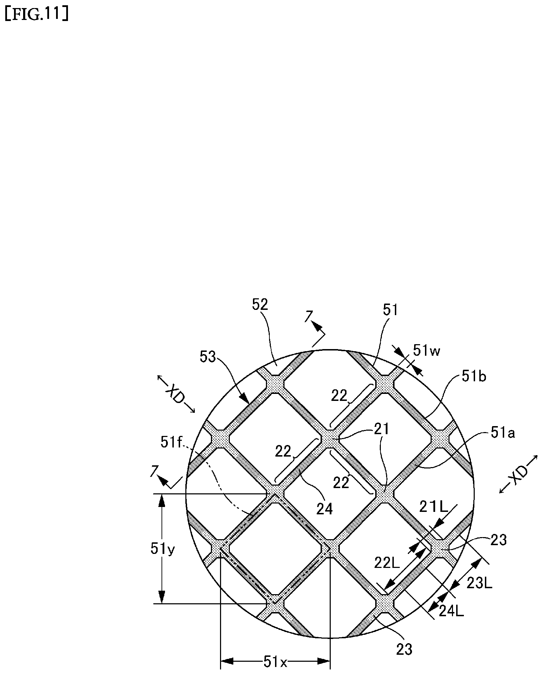

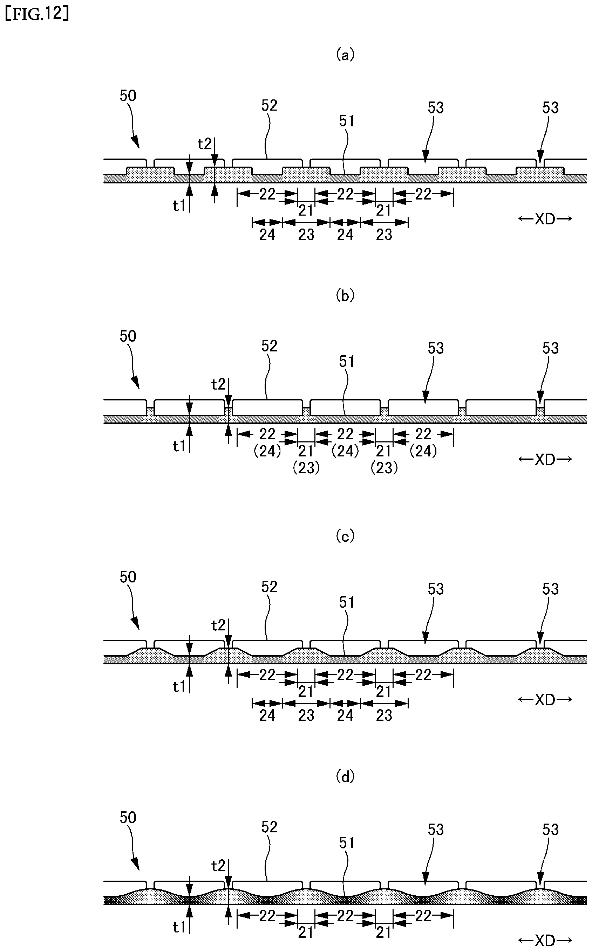

The bottom parts 51 of the grooves 53 formed in the lattice pattern in the top face of the absorbent element 50 have crossing zones 21 and non-crossing zones 22 each located between two adjacent crossing zones 21, and at least partly over the top face of the absorbent element 50, the thickness of the bottom part 51 is preferably larger stepwise or continuously from the middle in the longitudinal direction (the direction XD of continuation of the groove 53) of a non-crossing zone 22 toward the adjacent crossing zones 21, as shown in Figs. 11 and 12. Note that the densely-dotted zones in the drawings represent relatively smaller thickness of the bottom parts 51 of the grooves 53 and thus relatively highly-compressed zones, whereas the thinly-dotted zones represent relatively larger thickness of the bottom parts 51 of the grooves 53 and thus relatively less-compressed zones.

In the embodiment shown in Fig. 12(a), the bottom part 51 of each non-crossing zone 22 has a first thickness t1, which is generally uniform, in the middle in the longitudinal direction, and the bottom part 51 of the remaining zones, i.e., the crossing zones 21 and regions of each non-crossing zone 22 closer to the adjacent crossing zones 21, have a second thickness t2, which is generally uniform and larger than the first thickness t1. In the embodiment shown in Fig. 12(b), the bottom part of each non-crossing zone 22 has a first thickness t1, which is generally uniform roughly entirely in the longitudinal direction, and the bottom part of each crossing zone 21 has a second thickness t2, which is generally uniform roughly entirely and larger than the first thickness t1. These embodiments are examples where the thickness of a bottom part 51 varies stepwise.

On the other hand, in the embodiment shown in Fig. 12(c), the bottom part 51 of each non-crossing zone 22 has a first thickness t1, which is generally uniform, in the middle in the longitudinal direction, and the bottom part 51 of each crossing zone 21 has a second thickness t2, which is generally uniform roughly entirely and larger than the first thickness t1, and the transition region between these has a continuously increasing thickness from the first thickness t1 to the second thickness t2 (sloped). In the embodiment shown in Fig. 12(d), the thickness of the bottom part 51 varies continuously in the longitudinal direction of each groove 53 such that the bottom part 51 of each non-crossing zone 22 has the smallest thickness (first thickness t1) in generally the middle in the longitudinal direction, and the bottom part 51 of each crossing zone 21 has the largest thickness (second thickness t2) in generally the middle (in a section of each groove 53 along the direction of its continuation, the contour of the bottom part of the groove 53 is sinusoidal). These embodiments are examples where the thickness of a bottom part 51 varies continuously.

In such bottom part-thickness-varying regions, (a) with the thickness of the bottom part 51 being larger stepwise or continuously from the middle in the longitudinal direction of each non-crossing zone 22 toward the adjacent crossing zones 21 (i.e., the degree of compression and the density of the bottom part 51 are lower from the middle in the longitudinal direction of each non-crossing zone 22 toward the adjacent crossing zones 21), decrease in thickness at a corner of each non-compressed area 52 facing a crossing zone 21 of the bottom parts 51 of the grooves 53 is reduced, so that the crossing zones 21 of the bottom parts 51 of the grooves 53 are hardly touched by the skin of the user. In this way, compared to the bottom part 51 of each groove having generally a uniform thickness, the grainy texture of the high-absorbent polymer particles is harder to be felt by the user. Further, (b) at each crossing zone 21 of the bottom parts 51 of the grooves 53, which is more likely to be touched by the kin of the user, increase in the amount of the high-absorbent polymer particles at or near the top face of the absorber body 56 is reduced, while densification and decrease in cushioning property in the thickness direction are also reduced, so that even when the crossing zones 21 of the bottom parts 51 of the grooves 53 are brought into contact with the skin of the user, the grainy texture of the high-absorbent polymer particles is slight and thus still harder to be felt by the user.

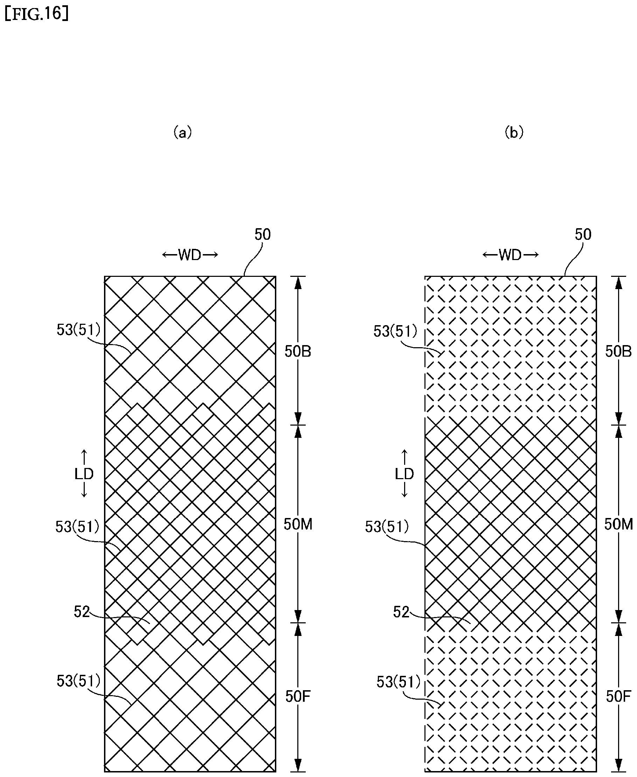

The bottom part-thickness-varying regions discussed above may be arranged all over the extent of the grooves 53 (i.e., all of the crossing zones 21 and the non-crossing zones 22 may be subjected to the thickness varying), or only partly over the extent of the grooves 53. In particular, taking into consideration the tendency of the grainy texture of the high-absorbent polymer particles to occur at a site subjected to external force, such as friction (the same is true for escape of the high-absorbent polymer particles through the surface of the wrapping nonwoven sheet 58), where the absorbent element 50 is divided into three sections 50C, 50L, 50R (e.g., divided into three equal sections) in the width direction WD as shown in Fig. 8, and the grooves 53 in the lattice pattern are formed over these three sections 50C, 50L, 50R, at least the sections 50L and 50R on opposed sides are preferably the bottom part-thickness-varying regions as discussed above. Further, taking into consideration of the fact that increased thickness of the bottom parts 51 in the crossing zones 21 adversely affects the liquid diffusibility or shape-retainability of the absorber body 56, the bottom parts 51 of all over the grooves 53 in the lattice pattern in the central section 50C preferably have the first thickness t1. Alternatively, with reference to the embodiments of Fig. 16 as will be discussed later, an embodiment is conceivable in which, while the central section 50C is the bottom part-thickness-varying region as discussed above, the grooves 53 are not crossed in the sections 50L, 50R on opposed sides (the absorbent element has no dents (zones compressed in the thickness direction) in the zones corresponding to the crossing zones 21), or an embodiment is also conceivable in which the area of each unit frame 51f of the grooves 53 is made larger (e.g., 1.5 times or more) in the sections 50L and 50R on the opposed sides than in the central section 50C. In these embodiments, the grainy texture of the high-absorbent polymer particles are particularly hard to be felt by the user, and the liquid diffusibility in the width direction WD is lower, leading to side leakage hardly occurred.

When each region 24 of the first thickness t1 and each region 23 of the second thickness t2 both have certain dimensions in the direction XD of continuation of a groove 53 (i.e., exclusive of embodiments having bottom parts 51 with continuously varying thickness), the dimension 23L of each region 23 of the second thickness t2 in the direction XD of continuation of a groove 53 is preferably 0.3 to 1 time, more preferably 0.5 to 0.9 times the dimension 22L in the longitudinal direction of a non-crossing zone 22. The dimension 24L of each region 24 of the first thickness t1 in the direction XD of continuation of a groove 53 is preferably 1 time or more, particularly 1.1 times or more the dimension 21L of each crossing zone 21 in the direction XD of continuation of a groove 53 (the distance between a pair of adjacent non-crossing zones 22 having a crossing zone 21 interposed therebetween), and preferably less than 0.75 times the dimension 22L in the longitudinal direction of a non-crossing zone 22.

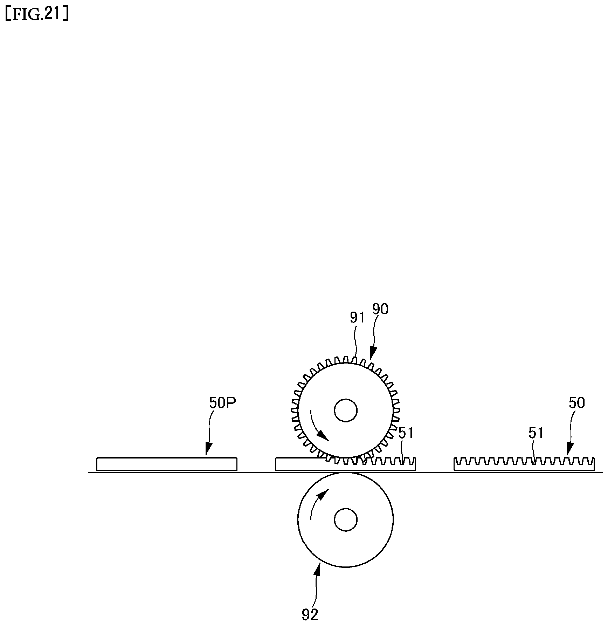

Such an absorbent element 50 may be produced by a commonly known method, such as embossing. For example, an absorbent element 50 having the grooves 53 may be manufactured through the first step of preparing an absorber body 56 by mixing and accumulating pulp fibers and high-absorbent polymer particles, the second step of preparing a wrapped body by wrapping the entirety of the absorber body 56 with a wrapping sheet 58, and the third step of passing the wrapped body 50P between an anvil roll 90 having on its roll surface a number of protrusions 91 arranged at intervals in a pattern corresponding to the pattern of the bottom parts 51 of the grooves 53, and a plain roll 92 having a cylindrical face (without protrusions 91) facing the anvil roll 90, as shown in Fig. 21, to compress the areas of the wrapped body 50P caught between the number of protrusions 91 on the anvil roll 90 and the plain roll 92, to thereby form the grooves 53 with the bottom parts 51 compressed in the thickness direction. The compressing may be performed under heating of either or both of the anvil roll 90 and the plain roll 92, or without heating. In the third step, only the areas of the body 50P that are caught between the number of protrusions 91 on the anvil roll 90 and the plain roll 92 may be compressed, or while the overall wrapped body 50P is compressed, the areas of the wrapped body 50P that are caught between the number of protrusions 91 on the anvil roll 90 and the plain roll 92 may be compressed into the deepest.

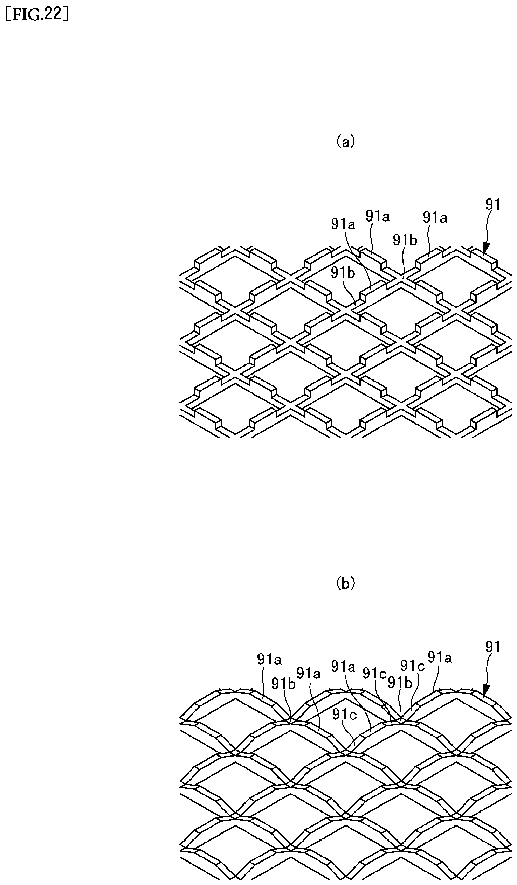

For forming the grooves 53 of the embodiment shown in Fig. 12(a), the pattern of the protrusions 91 on the anvil roll 90 is as shown in Fig. 22(a), whereas for forming the grooves 53 of the embodiment shown in Fig. 12(c), the pattern of the protrusions 91 on the anvil roll 90 is as shown in Fig. 22(b). By depressing with first protrusions 91a on the anvil roll 90, the bottom parts 51 with the first thickness t1 are formed, and by depressing with second protrusions 91b on the anvil roll 90, the bottom parts 51 with the second thickness t2 are formed. Further, with each inclined protrusion 91c connecting a first protrusion 91a and a second protrusion 91b, an inclined bottom part 51 is formed connecting a bottom part 51 of the first thickness t1 and a bottom part 51 of the second thickness t2.

The thickness 50t of the absorbent element 50 and the thickness 51t (t1, t2) of the bottom part 51 of each groove may suitably be decided and, for example, the maximum value of the thickness 50t of the absorbent element 50 may be preferably 4 to 35 mm, particularly preferably 5 to 13 mm (maximum thickness of the non-compressed areas 52). The first thickness t1 of the bottom parts 51 may suitably be decided and, generally preferably 15 to 35% the maximum value of the thickness 50t of the absorbent element 50. The second thickness t2 of the bottom parts 51 is preferably 1.4 to 6 times, more preferably 2 to 4 times the first thickness t1.

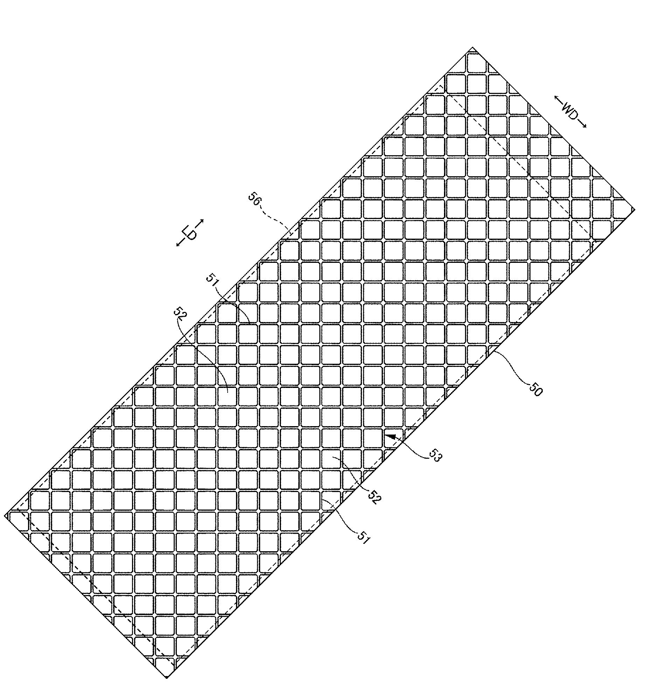

One preferred pattern of the grooves 53 is a rhombic lattice pattern composed of first lines 51a, each extending obliquely at 40 to 50° clockwise with respect to the front-back direction LD in plan view, and second lines 51b, each extending obliquely at 40 to 50° counterclockwise with respect to the front-back direction LD in plan view, as in the embodiment shown in Figs. 10 and 11. In this embodiment, the unit frame 51f has a generally diamond shape. The dimensions of the unit frame 51f may suitably be decided, and the dimension 51x in the width direction WD of the unit frame 51f may be about 15 to 20 mm. The dimension 51y in the front-back direction LD of the unit frame 51f may be about 15 to 20 mm.

The width 51w of the bottom part 51 of each groove 53 may suitably be decided, and usually preferably about 1 to 3 mm and preferably generally uniform in the direction of continuation of a groove 53.

The area percentage of the bottom parts 51 of the grooves 53 in the top face of the absorbent element 50 may suitably be decided, but may preferably be about 10 to 14% since excessively dense arrangement of the bottom parts 51 of the grooves 53 may make the absorbent element 50 too stiff.

As in the illustrated embodiments, it is preferred to make each corner of each crossing zone 21 in a non-compressed area 52 has a plurality of vertices to reduce the angle of each vertex and, in this case, a crossing zone 21 refers to the closed zone formed by connecting all the vertices (the octagonal zone in the illustrated embodiment). Similarly, each corner of each crossing zone 21 in a non-compressed area 52 may be rounded in an arc shape (not shown) and, in this case, a crossing zone 21 refers to the closed zone formed by connecting arc end points facing each other across a groove.

The section having the grooves 53 extending continuously in a lattice pattern may be arranged all over the top face of the absorbent element 50, or only partly. The continuous extension of the grooves 53 in a lattice pattern improves the liquid diffusibility but, placing such a section in the front or back end section of the absorbent element 50 may result in likelihood of front or back leakage. Thus, as shown, for example, in Fig. 16(a), it is preferred to divide the absorbent element into three sections, i.e., an intermediate section 50M located in the middle in the front-back direction LD and containing the crotch section M, and front section 50F forward of and back section 50B backward of the intermediate section 50M, and the grooves 53 extending continuously in a lattice pattern are provided all over these sections, with the area of each unit frame 51f in the front section 50F and the back section 50B being larger (e.g., about 1.5 to 3 times) than the area of each unit frame 51f in the intermediate section 50M. Alternatively, as shown in Fig. 16(b), it is also preferred that the grooves 53 extending continuously in a lattice pattern are provided in the intermediate section 50M, while in the front section 50F and the back section 50B, the grooves 53 are provided, but without crossing (the absorbent element has no dents (zones compressed in the thickness direction) in the zones corresponding to the crossing zones 21).

### <Wrapping Nonwoven Sheet>

The wrapping nonwoven sheet 58 is preferably nonwoven fabric having a meltblown layer formed of fibers having an average fiber diameter of 5 µm or less, and having an air permeability of 25.5 to 70.0 cm³/cm²·s, as determined of a stack of five sheets thereof by Method A (Frazier Type Method) in compliance with JIS L 1096: 2010. The air permeability of the wrapping nonwoven sheet 58 measured in the form of a stack of five sheets thereof is more preferably 40.0 to 70.0 cm³/cm²·s. This air permeability may be determined using, for example, an air permeability tester (Model AP-500KZ4) manufactured by DAIEI KAGAKUSEIKI MFG. CO., LTD. The meltblown layer is, as well known in the art, a nonwoven layer in which ultrafine fibers obtained by spinning a molten resin raw material in a high-velocity hot airstream are accumulated and subjected to interfiber bonding, and its constituent fibers have an average fiber diameter of as small as 10 µm or less, a fiber length of 30 µm or longer, which is referred to as long fibers, and have small interfiber gaps. The average fiber diameter of the constituent fibers of the meltblown layer is preferably 2.5 µm or smaller. On the other hand, the average fiber diameter of the constituent fibers of the meltblown layer may be 1 µm or larger, and preferably 2 µm or larger.

The total basis weight of the meltblown layer (where the wrapping nonwoven sheet has a plurality of meltblown layers, the sum of the basis weights of all the layers) and the total thickness of the meltblown layer (where the wrapping nonwoven sheet has a plurality of meltblown layers, the sum of the thicknesses of all the layers) may suitably be decided, and the former may be 0.3 to 6 g/m² and the latter may be 0.01 to 0.35 mm.

The wrapping nonwoven sheet 58 may be made, as far as including a meltblown layer, of a single-layer nonwoven fabric made solely of a meltblown layer, but may preferably be made of a composite nonwoven fabric having one or a plurality of meltblown layers and one or a plurality of protective layers. The composite nonwoven fabric may be manufactured by forming webs each constituting each layer, one on top of the other, and subjecting the stacked webs to thermal bonding (point bonding, hot-air bonding, or the like) to achieve the interfiber bonding and interlayer bonding, or by forming each nonwoven layer constituting each layer individually (formation of a web and interfiber bonding), and then stacking and bonding the obtained nonwoven layers thermally or with an adhesive.

The protective layer may preferably be a spunbonded layer. The spunbonded layer is, as well known in the art, a nonwoven layer in which filaments obtained by extruding (spinning) a molten resin raw material through nozzles are accumulated and subjected to interfiber bonding, and its constituent fibers have a larger average fiber diameter and also a longer fiber length, compared to the constituent fibers of the meltblown layer. Specifically, the wrapping nonwoven sheet 58 may preferably be made of SMS or SSMMS nonwoven fabric having at least one meltblown layer interposed between a pair of top and under spunbonded layers. The material of the constituent fibers of each layer is not particularly limited, and may be, for example, polypropylene fibers, polyethylene/polypropylene bicomponent fibers, or the like for the meltblown layers, and polypropylene fibers, polyethylene/polypropylene bicomponent fibers, or the like for the spunbonded layers.

Regarding the protective layer, the fiber diameter of the constituent fibers, the total basis weight (where the wrapping nonwoven sheet has a plurality of protective layers, the sum of the basis weights of all the layers), and the total thickness (where the wrapping nonwoven sheet has a plurality of protective layers, the sum of the thicknesses of all the layers) may suitably be decided. For example, the constituent fibers of the protective layer preferably have an average fiber diameter of 10 to 25 µm (particularly 15 to 20 µm). The total basis weight of the protective layer is preferably 5 to 17.5 g/m² (particularly 5 to 10 g/m²), and the total thickness is preferably 0.01 to 0.35 mm (particularly 0.1 to 0.2 mm).

The basis weight and the thickness of the wrapping nonwoven sheet 58 may suitably be decided. For example, the total basis weight of the wrapping nonwoven sheet 58 is preferably 10 to 17 g/m², and the thickness of the wrapping nonwoven sheet 59 is preferably 0.2 to 0.8 mm.

In the absence of stretchability of the wrapping nonwoven sheet 58, grooves of a sound structure are hard to be formed, and flexibility of the absorbent element 50 is also low. In this light, the wrapping nonwoven sheet 58 preferably has an elongation of 20 to 100%, particularly 30 to 60%, in the front-back direction LD under normal conditions as determined in compliance with JIS L 1913: 2010, and an elongation of 20 to 110%, particularly 50 to 70% in the width direction WD under normal conditions as determined in compliance with JIS L 1913: 2010. With uniform grooves formed using such nonwoven fabric, the grainy texture of the high-absorbent polymer particles is readily felt by the user. However, with the bottom part-thickness-varying region as discussed above, the absorbent element may have improved shape-retainability and flexibility of the absorber body 56, while the grainy texture of the high-absorbent polymer particles may be kept from being felt by the user.

Further, where nonwoven fabric is used as the wrapping nonwoven sheet 58, which has one or a plurality of meltblown layers formed of fibers having an average fiber diameter of 5 µm or smaller and one or a plurality of protective layers formed of fibers having an average fiber diameter of 10 to 25 µm, and has the elongation discussed above, it is preferred to employ the basis weight of 15 to 17 g/m² of the wrapping nonwoven sheet 58 and the bottom part-thickness-varying region as discussed above, so that the high-absorbent polymer particles hardly escape through the wrapping nonwoven sheet 58. In other words, where the bottom part 51 thickness is larger stepwise or continuously from the middle in the longitudinal direction of each non-crossing zone 22 toward the adjacent crossing zones 21 (that is, the degree of compression and the density of the bottom part 51 are lower from the middle in the longitudinal direction of each non-crossing zone 22 toward the adjacent crossing zones 21), compared to otherwise, elongation of the wrapping nonwoven sheet 58 along both sides of each groove 53, as well as the resulting enlargement of the interfiber gaps and decrease in thickness, may be reduced from the middle in the longitudinal direction of each non-crossing zone 22 toward the adjacent crossing zones 21. In this way, by combining a dense wrapping nonwoven sheet 58 having a basis weight higher than a certain level, and the varying thickness of the bottom part 51 as discussed above, the high-absorbent polymer particles contained in the absorber body 56 hardly escape through the wrapping nonwoven sheet 58 even in or near the crossing zones 21.

How to wrap the absorber body with the wrapping nonwoven sheet 58 may suitably be decided and, in view of readiness of production or protection against leakage of the high-absorbent polymer particles through the front or back end edge of the resulting wrapped body, preferably, as in the illustrated embodiment, the wrapping nonwoven sheet 58 is wrapped cylindrically around the absorber body 56 to surround its top and under faces as well as both side faces, with the front and back edge portions of the wrapping nonwoven sheet extending forwardly and backwardly beyond the absorber body 56, and the overlapped areas and the front and back extensions are preferably joined with joining means, such as a hot melt adhesive or material melt-bonding.

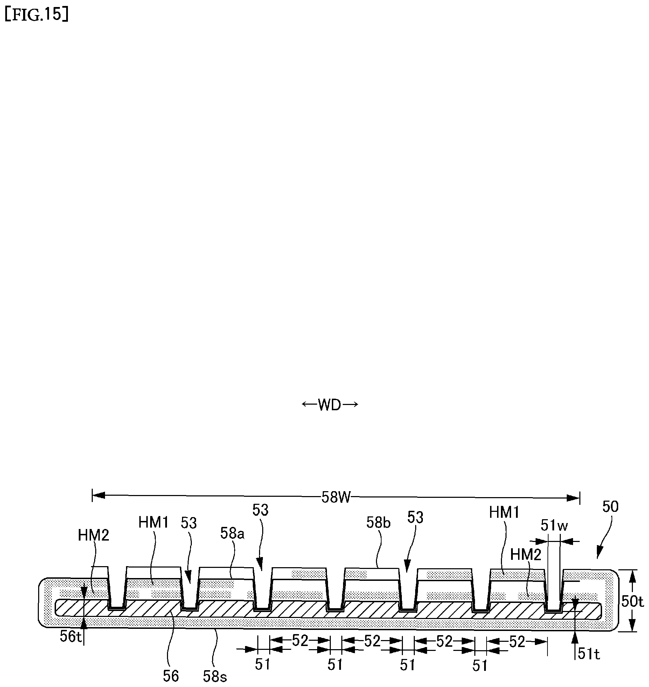

In particular, as shown in Fig. 15, the wrapping nonwoven sheet 58 has an under section 58s located on the underside of the absorber body 56, a first top section 58a extending continuous from the under section 58s and around a side edge to the top side of the absorber body 56, and a second top section 58b extending continuous from the under section 58s and around the other side edge to the underside of the absorber body 56, and the first top section 58a and the second top section 58b overlap with each other in a layered section 58W, wherein all of the grooves are formed. In this way, the high-absorbent polymer particles may preferably be kept from escaping through the top face of the absorbent element 50.

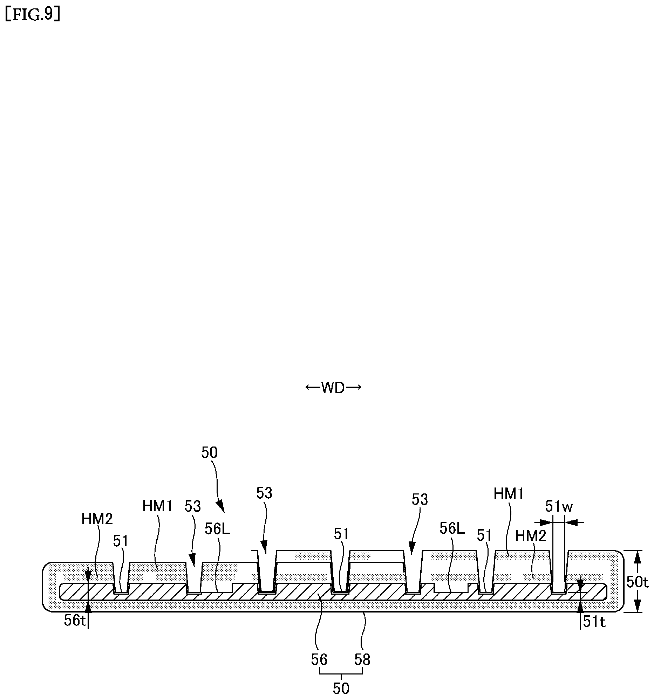

The absorbent element 50, for cost reduction, may not at all have any other sheet layer, such as of paper or nonwoven fabric (but may necessarily have an adhesive layer), between the wrapping nonwoven sheet 58 and the absorber body 56 as shown in Fig. 9 or the like, or may have such other sheet layer on at least one of the top and under sides of the absorber body 56.

### <Standup Gather Parts>

Each of the standup gather parts 60 has a standup zone 68 which stands up from the inner member 200 on a lateral side thereof, and is brought into contact with the wearer from his/her groin along the round-leg profile up to the buttocks to avoid side leakage. The standup gather parts 60 in the illustrated embodiment each has a root-side portion 60B obliquely standing up toward the center in the width direction, and a distal-side portion 60A than the intermediate portion obliquely standing up outwards in the width direction. However, the standup gather parts are not limited to this, and suitable modification may be made, so that, for example, each of the gather part as a whole may stand up toward the center in the width direction.

More specifically, each of the standup gather parts 60 in the illustrated embodiment is composed of a strip-like gathered sheet 62 which is of the length equal to the front-back dimension of the inner member 200, and is folded in double at the distal portion in the width direction WD to form a distal edge along the folded line, and a plurality of elongate gathering elastic members 63 held between the layers in the folded area and the vicinity thereof and arranged at intervals in the width direction WD, with each of the gathering elastic members 63 being in its stretched state in the longitudinal direction. The proximal portion located opposite from the distal portion of each standup gather part 60 (the edge portion opposite in the width direction WD from the folded-edge portion of the sheet) is referred to as a root portion 65, which is fixed to a lateral side of the inner member 200, and the portion other than the root portion 65 is referred to as a main body portion 66 (the portion on the folded-edge-portion side) extending from the root portion 65. The main body portion 66 has the root-side portion 60B extending toward the center in the width direction, and a distal-side portion 60A folded along the distal edge of the root-side portion 60B and extending outwards in the width direction. Front and back end zones of each main body portion 66 are fixed in a laid-down state onto the top sheet 30 on one of its opposed sides to form laid-down zones 67, while the middle zone in the front-back direction between these zones is a non-fixed standup zone 68, wherein at least the distal portion thereof contains gathering elastic members 63 in a stretched state, each extending along the front-back direction LD.

The standup zone 68 of each of the standup gather parts 60 configured in this way, stands up to abut the skin as shown by the arrow in Fig. 3 by means of the contraction force of the gathering elastic members 63. In particular, with the root portions 65 positioned on the underside of the inner member 200, the standup zones 68 stand up to open outwards in the width direction in and near the crotch section, so that the standup gather parts 60 are brought into surface contact with around the legs to improve fitting. Alternatively, the root portions 65 may be fixed on the top side of the inner member 200, i.e., on the top face of the top sheet 30 on its lateral sides.

As with the standup gather parts 60 of the illustrated embodiment, when the main body portion 66 has a bent structure composed of the root-side portion 60B extending toward the center in the width direction and a distal-side portion 60A folded along the distal edge of the root-side portion 60B and extending outwards in the width direction, the distal-side portion 60A and the root-side portion 60B are joined together in the laid-down state, and the root-side portion 60B is joined in the laid-down state to the top sheet 30, in the laid-down zones 67. For the joining between the facing surfaces in the laid-down zones 67, at least one of a hot melt adhesive in various application manners and means of material melt-bonding, such as heat sealing and ultrasonic sealing, may be employed. In this case, the joining between the root-side portion 60B and the top sheet 30 and the joining between the distal-side portion 60A and the root-side portion 60B may be established by same or different means. For example, it is preferred to establish the joining between the root-side portion 60B and the top sheet 30 with a hot melt adhesive, and the joining between the distal-side portion 60A and the root-side portion 60B by material melt-bonding.

The gathered sheets 62 may preferably be made of nonwoven fabric having flexibility and superior in uniformity and concealability, such as spunbonded nonwoven fabric (SS, SSS, or the like), SMS nonwoven fabric (SMS, SSMMS, or the like), or meltblown nonwoven fabric, optionally subjected to water-repellent treatment with silicone or the like, as necessary. In this case, the fiber basis weight of the nonwoven fabric may preferably be about 10 to 30 g/m². Further, though not shown, a waterproof film may be interposed between the layers of the double-folded gathered sheet 62.

The gathering elastic members 63 may be rubber threads. When Spandex rubber threads are to be used, the fineness thereof may be preferably 470 to 1240 dtex, more preferably 620 to 940 dtex. The stretch rate of the gathering elastic members 63 in the fixed state may be preferably 150 to 350%, more preferably 200 to 300%. The number of the gathering elastic members 63 may be preferably two to six, more preferably three to five. The interval of the gathering elastic members 63 may appropriately be 3 to 10 mm. In this way, the region containing the gathering elastic members 63 is easily brought into surface contact with the skin. The gathering elastic members 63 may be arranged not only in the distal side, but also in the root side.

In the standup zone 68 of the standup gather part 60, at least one of a hot melt adhesive in various application manners and fixing means of material melt-bonding, such as heat sealing and ultrasonic sealing, may be employed for adhering the inner and outer layers of the gathered sheet 62 together or fixing the gathering elastic members 63 therebetween. If the inner and outer layers of the gathered sheet 62 are bonded all over, flexibility of the sheet is impaired, so that it is preferred not to bond or to bond weakly the areas of the inner and outer layers other than the bonding areas of the gathering elastic members 63. In the illustrated embodiment, by applying a hot melt adhesive only to the circumferential surfaces of the gathering elastic members 63 using application means, such as comb guns or surewrap nozzles, and holding the gathering elastic members 63 between the inner and outer layers of the gathered sheet 62, not only the fixing of the gathering elastic members 63 to the inner and outer layers of the gathered sheet 62, but also the fixing between the inner and outer layers of the gathered sheet 62, may be achieved by means of only the hot melt adhesive applied on the circumferential surfaces of the gathering elastic members 63.

Similarly, for fixing the laid-down zones 67, at least one of a hot melt adhesive in various application manners and means of material melt-bonding, such as heat sealing and ultrasonic sealing, may be employed.

### <Side Flap>

As shown in Figs. 1 to 4, the inner member 200 is provided, on its opposed lateral sides, side flaps 70 extending laterally outwards of the absorber body 56, and each side flap 70 preferably has a side stretchable zone SG stretchable in the front-back direction. Each side flap 70 in the illustrated embodiment has one or a plurality of elongate side elastic members 73 arranged at intervals, each extending in the front-back direction LD, a first sheet layer 71 facing the external faces of the side elastic members 73, and a second sheet layer 72 facing the internal faces of the side elastic members 73.

The first sheet layer 71 and the second sheet layer 72 may be made of any sheet material without particular limitation, and suitable nonwoven fabric, such as those usable for the standup gather parts 60 or the outer members 12F, 12B, may be selected. In the illustrated embodiment, the gathered sheet 62 of each standup gather parts 60 is extended to form the first sheet layer 71 and the second sheet layer 72. In this case, the front and back ends of the side flaps 70 are coincident with the front and back ends of the standup gather parts 60 (i.e., in this case, the front and back ends of the inner member 200), as will be discussed later.

The side elastic members 73 are not particularly limited, and elongate elastic members similar to the gathering elastic members 63 may be used. The stretch rate of the side elastic members 73 in the fixed state may be preferably 150 to 350%, more preferably 200 to 270%. The number of the side elastic members may be preferably two to sixteen, more preferably six to ten. The interval of the side elastic members may appropriately be 5 to 10 mm.

The side elastic members 73 are fixed to the first sheet layer 71 and the second sheet layer 72. For adhering the first sheet layer 71 and the second sheet layer 72 together or fixing the side elastic members 73 therebetween, a hot melt adhesive HM in various application manners or fixing means of material melt-bonding, such as heat sealing and ultrasonic sealing, may be employed. If the joined area between the first sheet layer 71 and the second sheet layer 72 is too large, flexibility of the sheets is impaired, so that it is preferred not to bond or to bond weakly the areas of the first and second sheet layers other than the bonding areas of the side elastic members 73. In the illustrated embodiment, by applying hot melt adhesive HM only to the circumferential surfaces of the side elastic members 73 using application means, such as comb guns or surewrap nozzles, and holding the side elastic members 73 between the first and the second sheet layers 71 and 72, not only the fixing of the side elastic members 73 to the first and the second sheet layers 71 and 72, but also the fixing between the first and the second sheet layers 71 and 72, may be achieved by means of only the hot melt adhesive HM applied on the circumferential surfaces of the side elastic members 73.

In the illustrated embodiment, the sheet material of the first sheet layer 71 and the sheet material of the second sheet layer 72 are folded along the side edge of the side flap 70, and the folded part is fixed on the under face of the liquid impervious sheet 11 (formed into a sealed bag). This fixing may be achieved with a hot melt adhesive HM as in the illustrated embodiment, or by material melt-bonding.

The side flaps may be omitted.

### <Outer Member>

The outer members 12F, 12B are composed of a rectangular front outer member 12F forming at least the lower torso section of the front body section F and a rectangular back outer member 12B forming at least the lower torso section of the back body section B, as in the illustrated embodiment, and the front outer member 12F and the back outer member 12B may be non-continuous on their crotch sides and spaced apart in the front-back direction LD (two-segmented outer member). Alternatively, though not shown, the front body section F and the back body section B may be continuous (integral outer member). The spaced distance 12d in the front-back direction in the two-segmented outer member may be, for example, about 40 to 60% the overall length Y. In the illustrated embodiment, the lower edges of the front outer member 12F and the back outer member 12B extend linearly along the width direction WD, but at least one of the lower edges of the front and back outer members 12F and 12B may be curved along the round-leg profile.

The underpants-type disposable diaper with the two-segmented outer member, wherein the inner member 200 is exposed between the front outer member 12F and the back outer member 12B, is preferably provided, on the under face of the inner member 200, with a covering nonwoven layer 13 extending from between the front outer member 12F and the inner member 200 to between the back outer member 12B and the inner member 200 so that a liquid impervious film 11 is not exposed on the under face of the inner member 200. The inner and outer faces of the covering nonwoven layer 13 may be adhered to the surfaces facing these faces, respectively, via a hot melt adhesive. The covering nonwoven layer 13 may be made of, for example, nonwoven fabric suitably selected from those forming the outer members 12F, 12B. Though not shown, the outer member may be continuous from the front body section F through the crotch section to the back body section B. In this case, the outer member not only has regions corresponding to the lower torso regions T, but also has regions corresponding to the intermediate sections L.

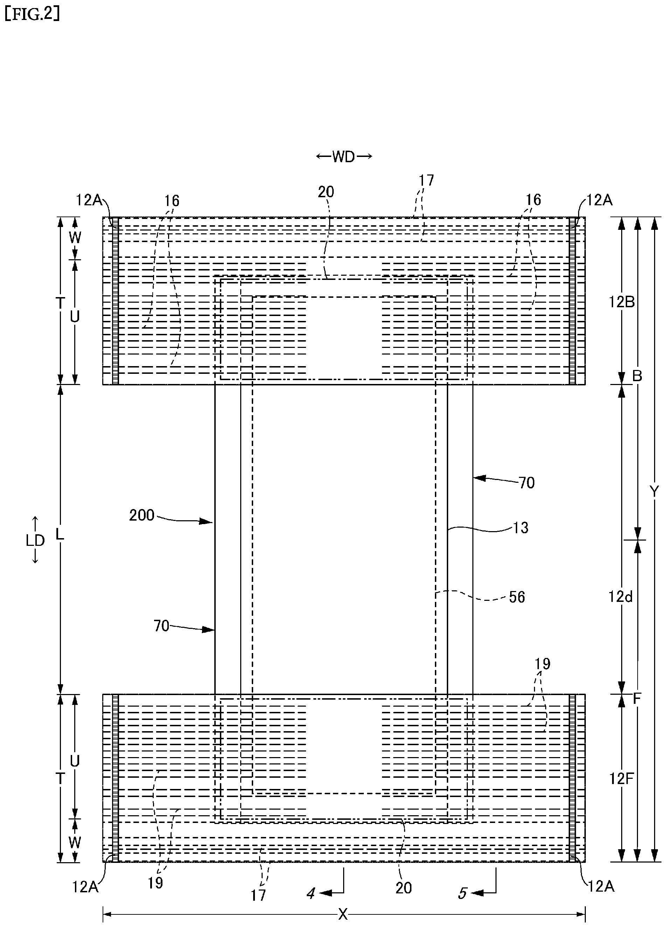

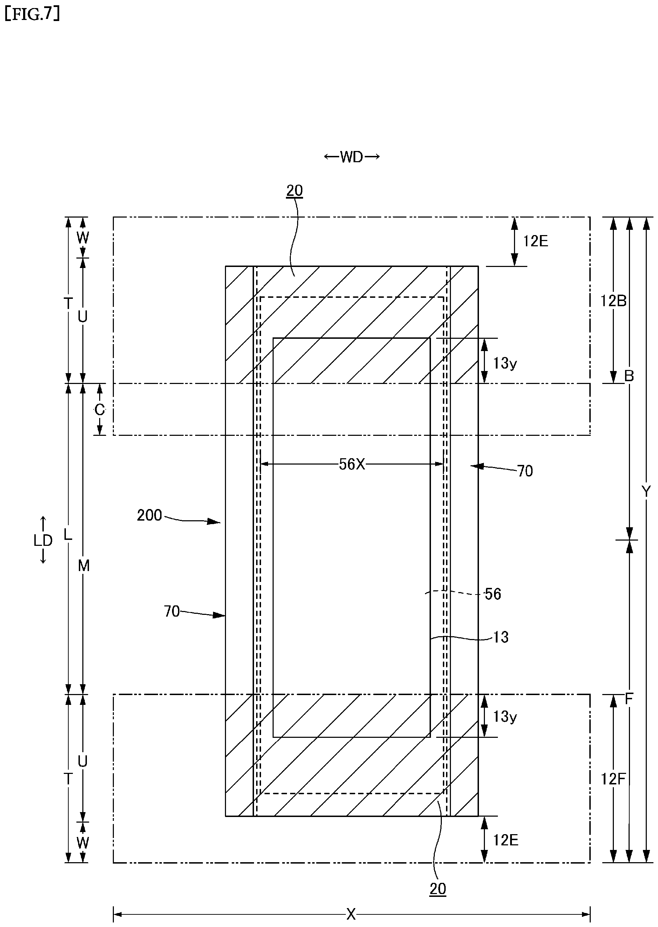

The front outer member 12F and the back outer member 12B have the front lower torso section and the back lower torso section, respectively, forming the lower torso regions T. In the embodiment shown in Figs. 1 and 2, the front outer member 12F and the back outer member 12B have the same dimension in the front-back direction LD, and neither the front outer member 12F nor the back outer member 12B has any zone corresponding to the intermediate region L. However, as shown in Fig. 7, the back outer member 12B may have a larger dimension in the front-back direction compared to the front outer member 12F, the front outer member 12F may have no zone corresponding to the intermediate region L, but the back outer member 12B may have a buttocks-covering zone C extending from the lower torso region T toward the intermediate region L. Though not shown, the front outer member 12F may also have a groin-covering zone extending from the lower torso region T toward the intermediate region L.

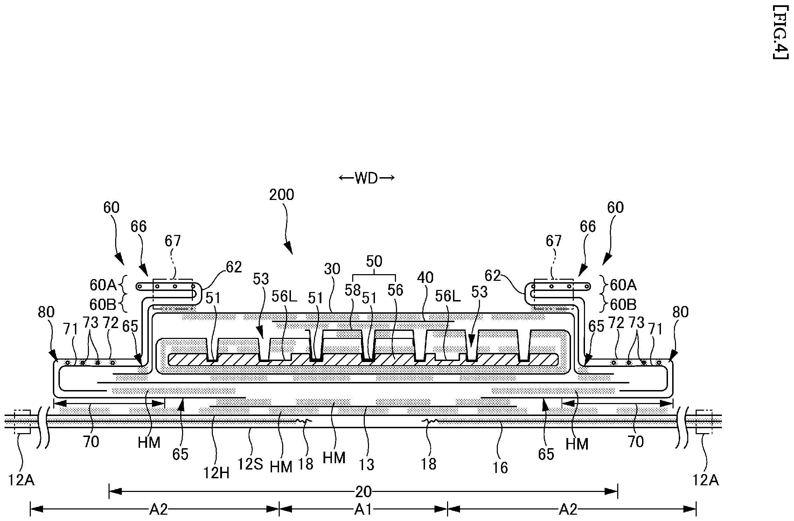

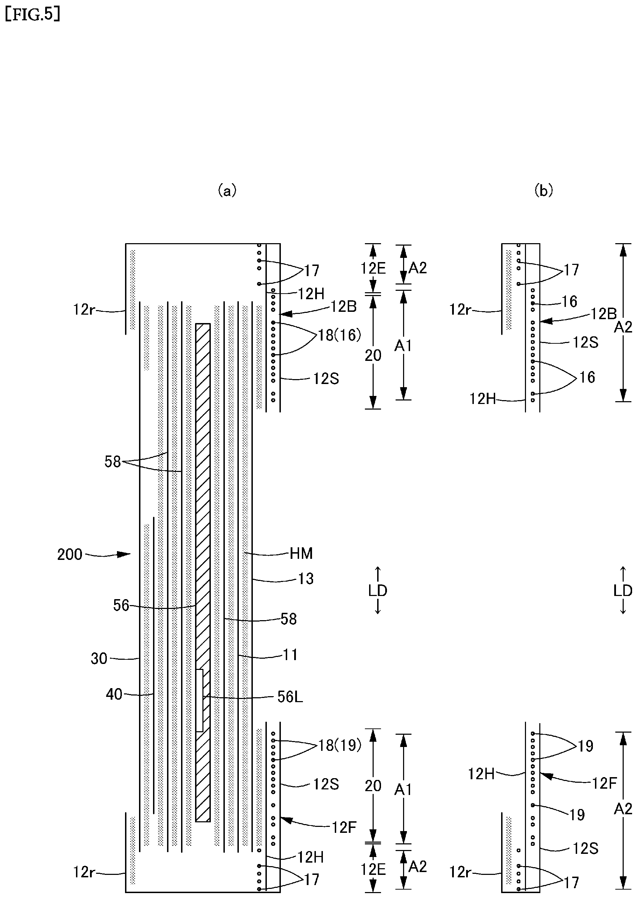

Each outer member 12F, 12B is formed by joining an outer sheet layer and an inner sheet layer adjacent outward and inward, respectively, to elastic members 16 to 19 to be discussed later, with joining means, such as a hot melt adhesive or melt-bonding, as shown in Figs. 4 and 5. The outer sheet layer and the inner sheet layer may be formed with two sheet materials 12S and 12H as in the illustrated embodiment, or may be formed with a single sheet material. For example, in the latter case, the inner sheet layer and the outer sheet layer may be formed with the inner section and the outer section, respectively, of a single sheet material folded along the edge of the waist opening WO (or the crotch-side edges) in part of or all over the outer members 12F, 12B. The illustrated embodiment represents the former, wherein the sheet material 12S forming the outer sheet layer in the under-waist zone is folded inward around the waist opening WO side of the sheet material 12H forming the inner sheet layer in the under-waist zone, and the resulting folded part 12r extends to cover the waist opening WO side end of the inner member 200. On the other hand, in the waist zone, the folded part 12r is the inner sheet layer adjacent inward to the elastic members.

For improved fitting on the lower torso of the wearer, each outer member 12F, 12B is provided with elastic members 16 to 19 therein to form a stretchable region A2, which is elastically stretchable in the width direction WD with the stretching/contracting of the elastic members 16 to 19. In this stretchable region A2, each of the outer members 12F, 12B in the natural length state is contracted with the contraction of the elastic members to form creases or ridges therein, and is stretchable in the longitudinal direction of the elastic members up to the predetermined stretch rate where the stretchable region is fully stretched without creases. The elastic members 16 to 19 may be elongate elastic members, such as rubber threads (as illustrated in the figures), or any conventionally known elastic members, such as of a tape, net, or film shape, without particular limitation. The elastic members 16 to 19 may either be made of synthetic rubber or natural rubber.

The elastic members 16 to 19 in the illustrated embodiment will now be discussed in further detail. In the waist zone W of each outer member 12F, 12B, a plurality of waist elastic members 17 is attached at intervals in the front-back direction, with each member 17 extending continuously all over the width direction WD. One or a plurality of the waist elastic members 17 arranged adjacent to the under-waist zone U may be arranged coincident with the inner member 200, or may be arranged on the opposed lateral sides in the width direction of the inner member 200, except for a middle region in the width direction thereof, which is overlapped with the inner member 200. As the waist elastic members 17, about two to fifteen, particularly about four to ten rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 2 to 12 mm intervals, particularly 3 to 7 mm intervals, to render the waist zone W to have a stretch rate in the width direction WD of preferably 150 to 400%, particularly about 220 to 320%. Further, the waist zone W may not necessarily contain the elastic members of the same fineness or the same stretch rate all over the front-back direction LD, and may contain elastic members having partially different finenesses or stretch rates.

In the under-waist zone U of each outer member 12F, 12B, a plurality of under-waist elastic members 16, 19, which is a plurality of elongate elastic members, is attached at intervals in the front-back direction, to form under-waist stretchable regions (regions containing the under-waist elastic members 16, 19). As the under-waist elastic members 16, 19, about five to thirty rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 1 to 15 mm intervals, particularly 3 to 8 mm intervals, to render the under-waist zone U to have a stretch rate in the width direction WD of preferably 200 to 350%, particularly about 240 to 300%. Further, the under-waist zone U may not necessarily contain the elastic members of the same fineness or the same stretch rate all over the front-back direction LD, and may contain elastic members having partially different finenesses or stretch rates.

When the elastic members 16, 19 are provided in a zone extending in the front-back direction and coincident with the absorber body 56 as in the under-waist zone U in the illustrated embodiment, in order to avoid contraction in the width direction WD of the absorber body 56 partially or entirely, it is preferred, as shown in Figs. 4, 5, 20, and others, that the middle region in the width direction of the under-waist zone U containing part or all of the part of the elastic members 16, 19 coincident with the absorber body 56 in the width direction WD is made a non-stretchable region A1, and the regions on the opposed lateral sides in the width direction thereof are made stretchable regions A2 (under-waist stretchable regions in the illustrated embodiment). The dimension in the width direction of each stretchable region A2 arranged on the opposed lateral sides in the width direction of the non-stretchable region A1 may be approximately constant in the front-back direction LD as in the illustrated embodiment, or may vary in the front-back direction LD, though not shown. The dimension in the width direction of each stretchable region A2 arranged on the opposed lateral sides in the width direction WD of the non-stretchable region A1 may either be approximately the same or different between the front body section F and the back body section B.

Such stretchable regions A2 and non-stretchable region A1 may be formed by attaching the elastic members 16 to 17, 19 between the inner sheet layer and the outer sheet layer, and finely cutting the elastic members 16, 19 by applying pressure and heat or by cutting at a single location in the middle in the width direction or almost all over the region to be the non-stretchable region A1, to thereby wreck the stretchability in the non-stretchable region A1, while the stretchability in the stretchable regions A2 is left intact. Naturally, unnecessary elastic members 18 are to be left in the non-stretchable region A1, which do not substantially contribute to the stretchability.

The sheet material for the inner sheet layer 12H and the outer sheet layer 12S may be any material without particular limitation, and may preferably be nonwoven fabric. The nonwoven fabric, where used, preferably has a basis weight of about 10 to 30 g/m² per sheet.

The elastic members 16 to 19 may be fixed to the outer members 12F, 12B by any commonly known manner. Similarly, the inner sheet layer and the outer sheet layer may be joined to each other by any commonly known manner. For example, in the regions of the outer members 12F, 12B containing the elastic members 16 to 19, by applying a hot melt adhesive HM only to the circumferential surfaces of the elastic members 16 to 19 using application means, such as comb guns or surewrap nozzles, and holding the elastic members between the inner sheet layer and the outer sheet layer, not only the fixing of the elastic members 16 to 19 to the inner and outer sheet layers, but also the fixing between the inner and outer sheet layers may be achieved by means of only the hot melt adhesive applied on the circumferential surfaces of the elastic members 16 to 19.

### <Inner Member Joint Area>

The inner member 200 may be fixed to the outer members 12F, 12B by joining means, such as material melt-bonding, including heat sealing or ultrasonic sealing, or with a hot melt adhesive. In the illustrated embodiment, the under face of the inner member 200 is fixed to the internal faces of the outer members 12F and 12B via a hot melt adhesive applied to the under face of liquid-impervious sheet 11 and root portion 65 of standup gather parts 60. This inner member joint area 20 joining the inner member 200 and the outer members 12F, 12B may be provided almost all over the overlapping area between the inner member and each outer member as shown in Fig. 2, for example, in the region except for the regions on the opposed lateral sides in the width direction of the inner member 200.

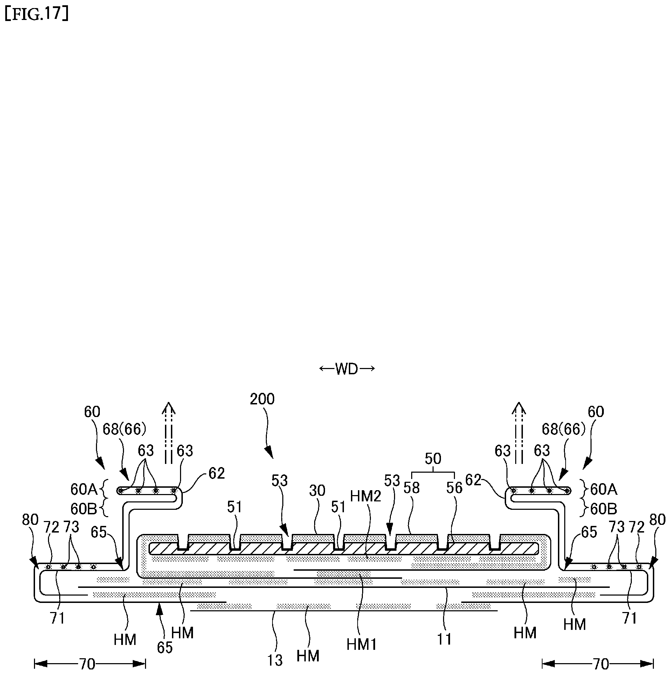

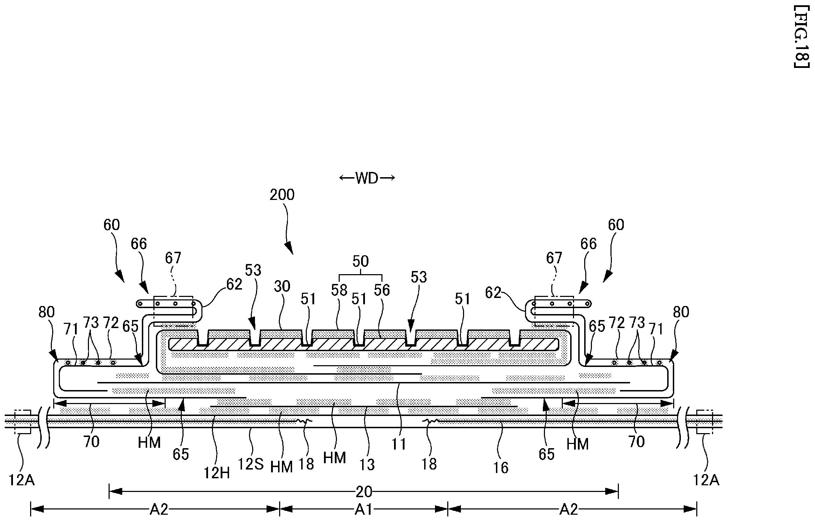

Since the high-absorbent polymer particles are kept from escaping through the wrapping nonwoven sheet 58 in the manner discussed above, members, such as the top sheet 30 covering the top face of the absorbent element 50 and the intermediate sheet 40, may be omitted as shown in Figs. 17 and 18, which allows significant cost reduction. In such an embodiment, as far as the middle region other than the opposed lateral sides in the width direction WD of the top face of the absorbent element 50 is devoid of any covering with another member all along the front-back direction LD to expose the wrapping nonwoven sheet 58 on the top face, only the middle region may be devoid of any covering with another member as in the illustrated embodiment, or the entirety of the top face of the absorbent element 50 may be devoid of any covering with another member.

### <Test for Confirming Effects>

Samples 1 to 8 of the underpants-type disposable diaper of the same structure as shown in Figs. 1 to 6 were fabricated, except that, as shown in Figs. 17 and 18, the top sheet 30 and the intermediate sheet 40 were omitted, and the width of the joining regions between the absorbent element 50 and the liquid-impervious sheet 11 were enlarged. The other specifications were as shown in Table 1. Specifications other than those shown in Table 1 were common to all Samples. Each of Samples 1 to 8 was tested for the grainy texture derived from the high-absorbent polymer particles, by lightly tracing the surface of a crossing zone 21 with a finger, and evaluated in two grades, i.e., evaluated as O when grainy texture was not felt or some particles were felt, or X when the grainy texture was felt. Further, Samples 1 to 8 were subjected to the test for confirming escape of the high-absorbent polymer particles as discussed below.

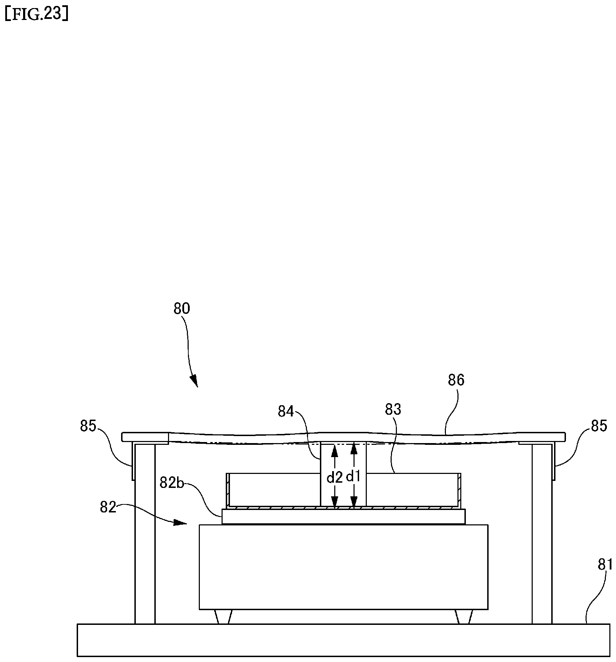

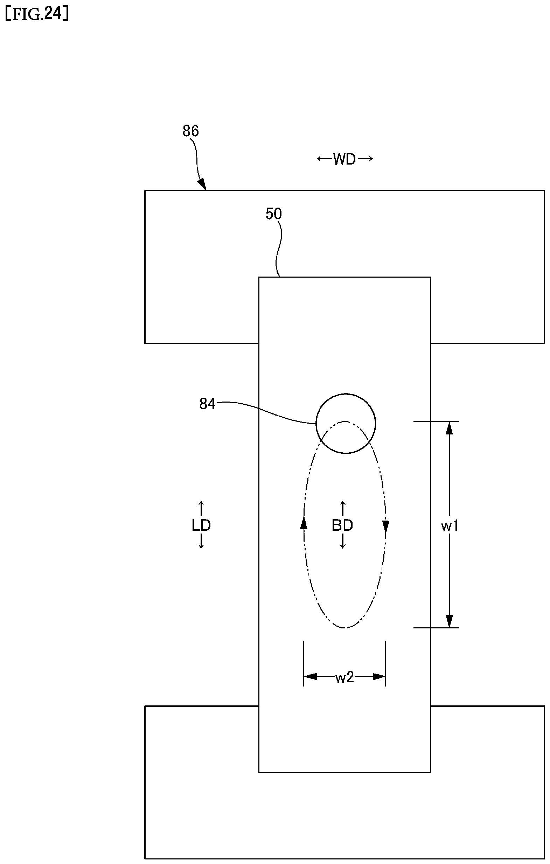

Fig. 23 shows test equipment 80. This test equipment 80 had a shaker 82 (model MK161 manufactured by YAMATO SCIENTIFIC CO., LTD.) placed on a horizontal plane 81, a collecting tray 83 fixed on a shaking stage 82b of the shaker 82 for receiving the high-absorbent polymer particles, and a friction body 84 made of a metallic column (55 mm in diameter) fixed erect in the center of the collecting tray 83, and was configured to shake the collecting tray 83 and the friction body 84 integrally with the shaking stage 82b. The vertical distance d1 between the top face of the friction body 84 and the top face of the shaking stage 82b was 72 mm. On the opposed lateral sides of the shaker 82 on the horizontal plane 81, a pair of stands 85 was fixed erect. A sample 86 was unfolded into a spread state (as shown in Fig. 1), stretched across the stands 85 with its top face (exposed surface of the wrapping nonwoven sheet) down, and fixed to the stands 85. Here, the front-back direction LD of the sample 86 was aligned to the long axis BD of the elliptical shaking motion. The vertical distance d2 between the top face of the shaking stage 82b and the area of top face of the sample 86 where the friction body is brought into contact was set to 71 mm, in the absence of the friction body 84, so that the top face of the sample 86 and the friction body 84 were brought into contact with each other. Then, the shaking motion of the shaker 82 was switched to the elliptical motion (amplitude w1 of shaking in the long axis direction was 30 mm, and the amplitude w2 of shaking in the short axis direction was 20 mm), the sample 86 was shaken at 90 rpm for 2 minutes to move and rub the friction body 84 in the elliptical motion against the top face of the sample 86 as shown in Fig. 24, and the number of the high-absorbent polymer particles fell onto the collecting tray 83 was counted.

The results of the test are shown in Table 1. The grainy texture and the escape of the high-absorbent polymer particles were observed in Samples 1 and 5, whereas the grainy texture and the escape of the high-absorbent polymer particles were not observed in Samples 2, 3, 4, 6, 7, and 8, which were, though, provided with the grooves extending continuously in a lattice pattern.

**Table 1**

| Sample No. | | 1 | 2 | 3 | 4 | 5 | 6 | 7 | 8 |
|---|---|---|---|---|---|---|---|---|---|
| Packing Nonwoven Sheet | Type | SMS Nonwoven | ← | ← | ← | ← | ← | ← | ← |
| | Fiber Bonding Method | Thermal Point Bonding | ← | ← | ← | ← | ← | ← | ← |
| | Air Permeability Determined by Method A (cm³/cm²/sec) | 25.9 | ← | ← | ← | 25.0 | ← | ← | ← |
| | Basis Weight (g/m²) | 15 | ← | ← | ← | 17 | ← | ← | ← |
| Absorber Body | Pulp Basis Weight (g/m²) | 158 | ← | ← | ← | ← | ← | ← | ← |
| | SAP Basis Weight (g/m²) | 163 | ← | ← | ← | ← | ← | ← | ← |
| | Pulp : SAP | 49:51 | ← | ← | ← | ← | ← | ← | ← |
| | Thickness (mm) | 4.29 | 4.30 | 4.30 | 4.30 | 4.29 | 4.30 | 4.30 | 4.30 |
| | Pattern in Plan | Figs. 10 and 11 | ← | ← | ← | ← | ← | ← | ← |
| | Angle of Intersection of Grooves | 90 degrees | ← | ← | ← | ← | ← | ← | ← |
| | Width of Bottom 51w (mm) | 1 | ← | ← | 1.5 | 1 | ← | ← | 1.5 |
| | Dimensions of Unit Frame | 12.89 mm × 12.89 mm | ← | ← | 19.34 mm × 19.34 mm | 12.89 mm × 12.89 mm | ← | ← | 19.34 mm × 19.34 mm |
| | Area Percentage of Bottom (%) | 13 | ← | ← | ← | ← | ← | ← | ← |
| Groove | Bottom Thickness Varying Pattern | Uniform | Fig. 12(a) | Fig. 12(a) | Fig. 12(a) | Uniform | Fig. 12(a) | Fig. 12(a) | Fig. 12(a) |
| | First Thickness t1 (mm) | 0.4 | 0.6 | 0.4 | 0.6 | 0.4 | 0.6 | 0.4 | 0.6 |
| | Second Thickness t2 (mm) | - | 2.1 | ← | ← | - | 2.1 | ← | ← |
| | Length of Region 24 with First Thickness (mm) | - | 5.9 | 4.9 | 10.8 | - | 5.9 | 4.9 | 10.8 |
| | Length of Region 23 with Second Thickness (mm) | - | 7.0 | 8.0 | 8.5 | - | 7.0 | 8.0 | 8.5 |
| Texture of Bottom of Groove in Crossing Zone | | × | ○ | ○ | ○ | × | ○ | ○ | ○ |
| Number of SAP Particles Escaped (Particle) | | 6 | 0 | 0 | 0 | 3 | 0 | 0 | 0 |

| | | | | | | | | | |
|---|---|---|---|---|---|---|---|---|---|
| *1: SAP stands for high-absorbent polymer particles. *2: Samples with second thickness t2 indicated as "-" have first thickness t1 all over bottom part 51 of each groove 53. | | | | | | | | | |

### <Explanation of Terms in the Specification>

The following terms appearing in the present specification shall have the following means unless otherwise specified herein.

- The "front-back direction" refers to the direction shown by the reference sign LD (longitudinal direction) in the figures, whereas the "width direction" refers to the direction shown by the reference sign WD (transverse direction) in the figures, and the front-back direction and the width direction are orthogonal to each other.
- The "top side" refers to the side, when the article is worn, closer to the skin of the wearer, whereas the "underside" refers to the side, when the article is worn, away from the skin of the wearer.
- The "top face" refers to the face, when the article is worn, closer to the skin of the wearer, whereas the "under face" refers to the face, when the article is worn, away from the skin of the wearer.
- The "stretch rate" refers to a value with respect to the natural length being 100%. For example, a 200% stretch rate is synonymous with stretch in two folds.
- The "artificial urine" is a mixture of 2 wt% urea, 0.8 wt% sodium chloride, 0.03 wt% calcium chloride dihydrate, 0.08 wt% magnesium sulfate heptahydrate, and 97.09 wt% ion-exchanged water.
- The "gel strength" is determined as follows. To 49.0 g of artificial urine, 1.0 g of high-absorbent polymer is added and stirred with a stirrer. The resulting gel is left in a chamber with constant temperature and humidity at 40 °C at 60% RH for 3 hours, and then the temperature is returned to the ordinary temperature. The gel strength is measured in a curd meter (Curdmeter-MAX ME-500 manufactured by I. techno Engineering).
- The "average fiber diameter" is determined by observing the subject layer under a microscope (optical microscope, SEM, or the like) at a magnification of 1000 folds, selecting thirty of the constituting fibers at random, subjecting the fibers to measurement of the fiber diameter (µm), and calculating the average of the measured diameters.
- The "basis weight" is determined as follows. A specimen or test piece is preliminarily dried, left in a laboratory or in apparatus under the standard conditions (23 ± 1 °C temperature and 50 ± 2% relative humidity in the testing location) until constant mass is attained. The preliminary drying refers to attaining constant mass from a specimen or test piece in the environment at a temperature of 100 °C. No preliminary drying may be performed on fibers with an official regain of 0.0%. From the test piece of the constant mass, a specimen of 100 mm × 100 mm size is cut out using a sampling template (100 mm × 100 mm). The weight of the specimen is measured and multiplied by 100 times to calculate the weight per 1 m², which is taken as the basis weight.
- The "thickness" of a thick member, such as the absorber body 56, the absorbent element 50, and the bottom part 51 of the grooves 53, is measured using a thickness meter manufactured by OZAKI MFG. CO., LTD. (PEACOCK, dial thickness gauge Model J-B (measurement range of 0 to 35 mm, circular terminal with measurement area of 20 mm in diameter, measuring force of about 3.0 N, pressure of about 30 KPa)), with the specimen and the thickness meter kept horizontal.
- The "thickness" of a thin sheet, such as nonwoven fabric, is automatically measured using an automatic thickness meter (KES-G5 handy compression tester program) under a load of 0.098 N/cm² with the compression area of 2 cm². Note that the "thickness" of the wrapping nonwoven sheet refers to its thickness at a site where the absorbent element 50 is the thickest.
- The "thickness of a layer" in nonwoven fabric is determined by observing a cross-section of the subject layer at a site where the absorbent element 50 is thickest under a microscope (optical microscope, SEM, or the like) at a magnification of 1000 folds, selecting five measurement positions at random, measuring the nominal thickness at each of the five measurement positions, and calculating the average thereof.
- The "area percentage" refers to the percentage of the area of the targets per unit area, and is obtained and expressed in percentage by dividing the sum of the areas of the targets (e.g., the bottom parts 51 of the grooves 53) in the entire objective region (e.g., the under face of the absorbent element 50) by the area of that objective region.
- The water absorption is determined in accordance with JIS K7223 - 1996 "Testing method for water absorption capacity of super absorbent polymers".
- The water absorption speed is defined as the "time spent until the end point is reached" in carrying out JIS K7224 - 1996 "Testing method for water absorption speed of super absorbent polymers" using 2 g of superabsorbent polymer and 50 g of saline.
- The "spread state" refers to the state in which an article is spread flatly without contraction (including any contraction, such as contraction by means of elastic members) or slack.
- The size or dimensions of each part refers to the size or dimensions not in the natural length state but in the spread state, unless otherwise specified.
- A test or measurement shall be, in the absence of description about environmental conditions, performed in a laboratory or in apparatus under the standard conditions (23 ± 1 °C temperature and 50 ± 2% relative humidity in the testing location).

### INDUSTRIAL APPLICABILITY

The present invention is applicable to disposable diapers, such as underpants-type disposable diapers, tape-type disposable diapers, and pad-type disposable diapers, as well as absorbent articles in general, such as sanitary napkins.

### DESCRIPTION OF REFERENCE SIGNS

11: liquid-impervious sheet
12A: side seal
12B: back outer member
12E: waist extension
12F, 12B: outer member
12F: front outer member
12S, 12H: sheet material
13: covering nonwoven layer
16, 19: under-waist elastic member
17: waist elastic member
18: unnecessary elastic member
200: inner member
201: inner member joint area
21: crossing zone
22: non-crossing zone
30: top sheet
40: intermediate sheet
50: absorbent element
51: bottom part
51f: unit frame
52: non-compressed area
53: groove
56: absorber body
56L: lower-basis-weight area
56c: covering layer
57: back flow protection layer
58: wrapping nonwoven sheet
60: standup gather part
60A: distal-side portion
60B: root-side portion
62: gathered sheet
63: gathering elastic member
67: laid-down zone
68: standup zone
70: side flap
71: first sheet layer
72: second sheet layer
73: side elastic member
90: anvil roll
91: protrusion
92: plain roll
A1: non-stretchable region
A2: stretchable region
B: back body section
C: buttocks-covering zone
F: front body section
HM: hot melt adhesive
HM1: first hot melt adhesive
HM2: second hot melt adhesive
L: intermediate region
LD: front-back direction
LO: leg opening
M: crotch section
SG: side stretchable zone
T: lower torso region
U: under-waist zone
W: waist zone
WD: width direction
WO: waist opening
t1: first thickness
t2: second thickness

## Claims

1. An absorbent article having a crotch section, and front and back sections extending forward of and backward of the crotch section, respectively, the absorbent article comprising:
an absorbent element comprising an absorber body arranged in a position including the crotch section, and a wrapping nonwoven sheet wrapping the absorber body,
wherein the absorber body has a layer formed by mixing and accumulating pulp fibers and high-absorbent polymer particles,
wherein the absorbent element has grooves extending continuously in a lattice pattern, each forming a dent from a top face of the absorbent element down into the absorber body and compressed in a thickness direction to form a bottom part, and
wherein the grooves have crossing zones, and non-crossing zones each located between two adjacent crossing zones, and at least partly over the top face of the absorbent element, a thickness of the bottom part is larger stepwise or continuously from a middle in a longitudinal direction of each non-crossing zone toward the adjacent crossing zones.

2. The absorbent article according to claim 1,
wherein a basis weight of the pulp fibers in the absorber body is 100 to 450 g/m²,
wherein a ratio by weight of the pulp fibers to the high-absorbent polymer particles in the absorber body is 30:70 to 70:30,
wherein a maximum thickness of the absorbent element is 4 to 35 mm,
wherein a thickness of the bottom part in the middle in the longitudinal direction of each non-crossing zone is 15 to 35% the maximum thickness of the absorbent element, and
wherein a thickness of the bottom part in each crossing zone is 1.4 to 6 times the thickness of the bottom part in the middle in the longitudinal direction of the non-crossing zone.

3. The absorbent article according to claim 1 or 2,
wherein a width of the bottom part is 1 to 3 mm, and
wherein a pattern of the grooves is a rhombic lattice pattern composed of first lines, each extending obliquely at 40 to 50° clockwise with respect to a front-back direction in plan view, and second lines, each extending obliquely at 40 to 50° counterclockwise with respect to the front-back direction in plan view.

4. The absorbent article according to claim 1 or 2,
wherein the wrapping nonwoven sheet comprises one or a plurality of meltblown layers, and one or a plurality of protective layers,
wherein a basis weight of each wrapping nonwoven sheet is 10 to 17 g/m²,
wherein constituent fibers of each protective layer have an average fiber diameter of 10 to 25 µm,
wherein constituent fibers of each meltblown layer have an average fiber diameter of 5 µm or smaller,
wherein the wrapping nonwoven sheet has an air permeability of 25.5 to 70.0 cm³/cm²·s, as determined of a stack of five sheets thereof by Method A (Frazier Type Method) in compliance with JIS L 1096: 2010,
wherein the wrapping nonwoven sheet has an elongation of 20 to 100% in the front-back direction under normal conditions as determined in compliance with JIS L 1913: 2010, and
wherein the wrapping nonwoven sheet has an elongation of 20 to 110% in a width direction under normal conditions as determined in compliance with JIS L 1913: 2010.

5. The absorbent article according to claim 4, wherein the basis weight of the wrapping nonwoven sheet is 15 to 17 g/m².

6. The absorbent article according to claim 1 or 2, wherein the absorber body has a covering layer in which only the pulp fibers are accumulated, to cover a top face of the absorber body.

7. The absorbent article according to claim 1 or 2,
wherein the wrapping nonwoven sheet has an under section located on an underside of the absorber body, a first top section extending continuous from the under section and around a side edge to a top side of the absorber body, and a second top section extending continuous from the under section and around another side edge to the top side of the absorber body,
wherein the first top section and the second top section overlap with each other in a layered section, and
wherein all of the grooves are formed in the layered section.

8. The absorbent article according to claim 5, wherein a middle region other than opposed lateral sides in the width direction of the absorbent element is devoid of any covering with another member all along the front-back direction to expose the wrapping nonwoven sheet on a top face of the absorbent article.
